# EUROPEAN PATENT APPLICATION

(11) **EP 1 580 273 A1**
(43) Date of publication of application: **28.09.2005**
(21) Application number: 04007278.7
(22) Date of filing: 26.03.2004
(51) Int. Cl.: C12N 15/63

(54) **Peptide-based method for monitoring gene expression in a host cell**

(71) Applicant: Friedrich-Alexander-Universität Erlangen-Nürnberg, 91054 Erlangen (DE)
(72) Inventor: Hillen, Wolfgang, Prof. Dr. rer. nat., 91080 Uttenreuth (DE); Berens, Christian, Dr., 91052 Erlangen (DE); Klotzsche, Marcus, 91056 Erlangen (DE)
(74) Representative: Vossius & Partner

(57) **Abstract**

The present invention relates a method for monitoring the expression level of a gene in a host cell by modulating the activity of a regulatory biomolecule, comprising the steps of: (a) transforming a cell expressing a regulatory biomolecule with a nucleic acid molecule comprising an open reading frame encoding an interaction partner of said biomolecule in expressible form, wherein (i) said regulatory biomolecule is either a nucleic acid binding molecule that effects its regulatory activity upon binding or an allosterically controlled ribonucleic acid molecule; and (ii) the interaction partner of the biomolecule is encoded by a nucleic acid molecule comprising: (1) a nucleic acid sequence encoding a tagged (poly)peptide, or (2) a nucleic acid sequence encoding a tagged (poly)peptide or a peptide tag, a selectable marker gene and additional nucleotide sequences for site specific, in-frame integration of said nucleic acid molecule into the coding sequence of at least one host (poly)peptide of interest, wherein said tag comprises the interacting residues of the interaction partner and (b) assessing the expression level of the gene. Furthermore, the present invention relates to a method of producing and/or selecting a compound capable of modulating the activity of a nucleic acid binding protein comprising the steps of: (a) conducting a selection of compounds with the nucleic acid binding target protein under conditions allowing an interaction of the compound and the nucleic acid binding protein; (b) removing unspecifically bound compounds; (c) detecting specific binding of compounds to the nucleic acid binding target protein; (d) expressing in a cell, the nucleic acid binding protein and providing in trans the coding sequence of at least one indicator gene, wherein said coding sequence is under control of the target sequence of the nucleic acid binding protein; (e) adding a candidate compound to the cell of step (d); (f) determining the amount or activity of the indicator protein, wherein a reduced or increased amount of indicator protein is indicative of compounds, capable of modulating the activity of the nucleic acid binding protein; (g) selecting compounds capable of modulating the activity of the nucleic acid binding protein. Moreover, the present invention relates to nucleic acid molecules, polypeptides, expression vectors, host cells, ensembles of host cells and a non-human animal comprising said host cells. Finally, the present invention relates to a kit comprising the nucleic acid molecule, the (poly)peptide, the vector, the host cell or the ensemble of host cells of the present invention in one or more containers.

SEQUENCE LISTING

<110>  Friedrich-Alexander-Universität Erlangen-Nürnberg

<120>  PEPTIDE-BASED METHOD FOR MONITORING GENE EXPRESSION IN A HOST CELL

<130>  H 1776 EP

<160>  14

<170>  PatentIn version 3.1

<210>  1

<211>  17

<212>  PRT

<213>  artificial sequence

<220>

<223>  /note="Description of artificial sequence: polypeptides with affinity to
the Tet repressor"

<400>  1

Met Trp Thr Trp Asn Ala Tyr Ala Phe Ala Ala Pro Ser Gly Gly Gly
1               5                   10                  15

Ser

<210>  2

<211>  16

<212>  PRT

<213>  artificial sequence

<220>

<223>  /note="Description of artificial sequence: polypeptides with affinity to
the Tet repressor"

<400>  2

Trp Thr Trp Asn Ala Tyr Ala Phe Ala Ala Pro Ser Gly Gly Gly Ser
1               5                   10                  15

<210>  3

<211>  12

<212>  PRT

<213>  artificial sequence

<220>

<223>  /note="Description of artificial sequence: polypeptides with affinity to
the Tet repressor"

<400>  3

Trp Thr Trp Asn Ala Tyr Ala Phe Ala Ala Pro Ser
1               5                   10


<210>  4

<211>  20

<212>  PRT

<213>  artificial sequence

<220>

<223>  /note="Description of artificial sequence: polypeptides with affinity to
the Tet repressor"

<400>  4

Ser Gly Gly Ala Trp Thr Trp Asn Ala Tyr Ala Phe Ala Ala Pro Ser
1               5                   10                  15


Gly Gly Gly Ser
            20


<210>  5

<211>  20

<212>  PRT

<213>  artificial sequence

<220>

<223>  /note="Description of artificial sequence: polypeptides with affinity to
the Tet repressor"

<400>  5

Ser Gly Gly Ala Trp Thr Trp Asn Ala Tyr Ala Phe Ala Ala Ala Ser
1               5                   10                  15


Gly Gly Gly Ser
            20


<210>  6

<211>  20

<212>  PRT

<213>  artificial sequence

<220>

<223>  /note="Description of artificial sequence: polypeptides with affinity to
the Tet repressor"

<400>  6

Ser Gly Gly Ala Trp Thr Trp Asn Ala Tyr Ala Phe Ala Ala Pro Ala
1               5                   10                  15

Gly Gly Gly Ser
            20

<210>  7

<211>  20

<212>  PRT

<213>  artificial sequence

<220>

<223>  /note="Description of artificial sequence: polypeptides with affinity to
the Tet repressor"

<400>  7

Ser Gly Gly Ala Trp Thr Trp Asn Ala Tyr Ala Phe Ala Ala Pro Ser
1               5                   10                  15

Gly Arg Gly Ser
            20

<210>  8

<211>  20

<212>  PRT

<213>  artificial sequence

<220>

<223>  /note="Description of artificial sequence: polypeptides with affinity to
the Tet repressor"

<400>  8

Ser Gly Gly Ala Trp Thr Trp Asn Ala Tyr Ala Phe Ala Ala Pro Ser
1               5                   10                  15

Asp Gly Gly Leu
            20

<210>  9

<211>  20

<212>  PRT

<213>  artificial sequence

<220>

<223>  /note="Description of artificial sequence: polypeptides with affinity to
the Tet repressor"

<400>  9

Ser Gly Gly Ala Trp Thr Trp Asn Ala Tyr Ala Phe Ala Ala Pro Ser
1               5                   10                  15

Asp Gly Gly Leu
            20

<210>  10

<211>  20

<212>  PRT

<213>  artificial sequence

<220>

<223>  /note="Description of artificial sequence: polypeptides with affinity to
the Tet repressor"

<400>  10

Ser Gly Gly Ala Trp Thr Trp Asn Ala Tyr Ala Phe Ala Ala Pro Ser
1               5                   10                  15

Gly Gly Gly Trp
            20

<210>  11

<211>  20

<212>  PRT

<213>  artificial sequence

<220>

<223>  /note="Description of artificial sequence: polypeptides with affinity to
the Tet repressor"

<400>  11

Ser Gly Gly Ala Trp Thr Trp Asn Ala Tyr Ala Phe Ala Ala Pro Ser
1               5                   10                  15

Gly Gly Cys Ser
            20

<210>  12

<211>  20

<212>  PRT

<213>  artificial sequence

<220>

<223>  /note="Description of artificial sequence: polypeptides with affinity to
the Tet repressor"

<400>  12

Ser Gly Gly Ala Trp Thr Trp Asn Ala Tyr Ala Phe Ala Ala Pro Ser
1               5                   10                  15

Gly Gly Asp Ser
            20

<210>  13

<211>  20

<212>  PRT

<213>  artificial sequence

<220>

<223>  /note="Description of artificial sequence: polypeptides with affinity to
the Tet repressor"

<400>  13

Ser Gly Gly Ala Trp Thr Trp Asn Ala Tyr Ala Phe Ala Ala Pro Ser
1               5                   10                  15

Gly Gly Arg Ser
            20

<210>  14

<211>  20

<212>  PRT

<213>  artificial sequence

<220>

<223>  /note="Description of artificial sequence: polypeptides with affinity to
the Tet repressor"

<400>  14

Ser Gly Gly Ala Trp Thr Trp Asn Ala Phe Ala Phe Ala Ala Pro Ser
1               5                   10                  15

Gly Gly Gly Ser
            20

## Description

The present invention relates a method for monitoring the expression level of a gene in a host cell by modulating the activity of a regulatory biomolecule, comprising the steps of: (a) transforming a cell expressing a regulatory biomolecule with a nucleic acid molecule comprising an open reading frame encoding an interaction partner of said biomolecule in expressible form, wherein (i) said regulatory biomolecule is either a nucleic acid binding molecule that effects its regulatory activity upon binding or an allosterically controlled ribonucleic acid molecule; and (ii) the interaction partner of the biomolecule is encoded by a nucleic acid molecule comprising: (1) a nucleic acid sequence encoding a tagged (poly)peptide, or (2) a nucleic acid sequence encoding a tagged (poly)peptide or a peptide tag, a selectable marker gene and additional nucleotide sequences for site specific, in-frame integration of said nucleic acid molecule into the coding sequence of at least one host (poly)peptide of interest, wherein said tag comprises the interacting residues of the interaction partner and (b) assessing the expression level of the gene. Furthermore, the present invention relates to a method of producing and/or selecting a compound capable of modulating the activity of a nucleic acid binding protein comprising the steps of: (a) conducting a selection of compounds with the nucleic acid binding target protein under conditions allowing an interaction of the compound and the nucleic acid binding protein; (b) removing unspecifically bound compounds; (c) detecting specific binding of compounds to the nucleic acid binding target protein; (d) expressing in a cell, the nucleic acid binding protein and providing in trans the coding sequence of at least one indicator gene, wherein said coding sequence is under control of the target sequence of the nucleic acid binding protein; (e) adding a candidate compound to the cell of step (d); (f) determining the amount or activity of the indicator protein, wherein a reduced or increased amount of indicator protein is indicative of compounds, capable of modulating the activity of the nucleic acid binding protein; (g) selecting compounds capable of modulating the activity of the nucleic acid binding protein. Moreover, the present invention relates to nucleic acid molecules, polypeptides, expression vectors, host cells, ensembles of host cells and a non-human animal comprising said host cells. Finally, the present invention relates to a kit comprising the nucleic acid molecule, the (poly)peptide, the vector, the host cell or the ensemble of host cells of the present invention in one or more containers.

Several documents are cited throughout the text of this specification. The disclosure content of the documents cited herein (including any manufacturer's specifications, instructions, etc.) is herewith incorporated by reference.

Despite tremendous advances in our comprehension of the molecular basis of infectious diseases or diseases such as cancer, substantial gaps remain both in our understanding of disease pathogenesis and in the development of effective strategies for early diagnosis and for treatment. Proteomic approaches to investigate disease will overcome some of the limitations of other approaches. The opportunities as well as the challenges facing disease proteomics are formidable. Particularly promising areas of research include: delineation of altered protein expression, not only at the whole-cell or tissue levels, but also in subcellular structures, in protein complexes and in biological fluids; the development of novel biomarkers for diagnosis and early detection of disease; and the identification of new targets for therapeutics and the potential for accelerating drug development through more effective strategies to evaluate therapeutic effect and toxicity.

Despite earlier predictions to the contrary, infectious diseases remain a leading cause of worldwide death. A complicating factor in therapy for infectious diseases is the development of resistance to commonly used drugs (for example, as has occurred in tuberculosis), which heightens the need for developing effective new therapies. Interest in the application of proteomics to microbiology goes back at least two decades, with the pioneering work of Fred Neidhardt to characterize protein expression patterns in *Escherichia coli* under different growth conditions (VanBogelen, R. A., Schiller, E. E., Thomas, R. D. & Neidhardt, F. C. Diagnosis of cellular states of microbial organisms using proteomics. Electrophoresis 20, 2149-2159 (1999)).

The complete sequencing of a number of microbial genomes has provided a framework for characterizing the role of various proteins and their relevance for pathogenic persistence or for disease progression. A crucial aspect of the continuing fight against pathogenic organisms is the question of whether or not it will be possible to identify those proteins within the pathogenic proteome, the expression of which is essential for the survival of the pathogen. Many of these proteins are likely to be tissue specific proteins because an optimal adaptation to the local conditions within the infected host is probably the most important prerequisite for survival of the pathogen. Due to their outstanding relevance for survival, these tissue-specific proteins are likely to be prime targets for drug development.

At present, the characterization of tissue or environment specific gene expression is primarily based on biochemical analysis of individual proteins or protein sets [see (Hanash, 2003) and (Jain, 2000) for reviews and references cited therein]. For proteome analysis, pathogenic microorganisms are often grown *in vitro* (in a chemostat, for example) under conditions known or assumed to mimic conditions in a host organism *[Streptococcus mutans -* (Len et al., 2003); *Salmonella typhimurium* - (Deiwick et al., 2002); *Pseudomonas aeruginosa* - (Guina et al., 2003); *Leishmania infantum* - (El Fakhry et al., 2002); *Mycobacterium tuberculosis -* (Rosenkrands et al., 2002)]. The quest for identifying new tissue- or disease-specific proteins is hampered by the fact that methods like tissue micro-dissection, Protein Chip Array technologies, MALDI-TOF mass spectrometry or mass spectrometric analysis of *in situ* tissue sections are time-consuming, expensive and technically demanding (Hanash, 2003; von Eggeling et al., 2000); they are also often limited by the presence of buffer components in biological samples, the heterogeneity of the protein sample or the lack of adequate amounts of sample for analysis. Tagging of all identified ORFs allows either separation of cells expressing a tagged protein by FACS from those not expressing a tagged protein and quantification in the case of highly-expressed or strongly-localized proteinsin the case of a GFP-tag (Huh et al., 2003) or a precise quantification of a tagged protein's expression level in the case of a TAP-tag (Ghaemmaghami et al., 2003). Thus, quantification and visual detection still requires the introduction of two independent tags per protein. While the *in vitro* manipulation of growth conditions allows the isolation of large amounts of protein samples, this must not faithfully represent the situation in a living host organism.

Thus, the technical problem underlying the present invention was to provide means and methods for identifying gene expression under specific growth conditions including tissue specific gene expression in a host cell or in an in vivo animal model for a disease. The solution to this technical problem is achieved by providing the embodiments characterized in the claims.

Accordingly, the present invention relates to a method for monitoring the expression level of a gene in a host cell by modulating the activity of a regulatory biomolecule, comprising the steps of: (a) transforming a cell expressing a regulatory biomolecule with a nucleic acid molecule comprising an open reading frame encoding an interaction partner of said biomolecule in expressible form, wherein (i) said regulatory biomolecule is either a nucleic acid binding molecule that effects its regulatory activity upon binding or an allosterically controlled ribonucleic acid molecule; and (ii) the interaction partner of the biomolecule is encoded by a nucleic acid molecule comprising: (1) a nucleic acid sequence encoding a tagged (poly)peptide, or (2) a nucleic acid sequence encoding a tagged (poly)peptide or a peptide tag, a selectable marker gene and additional nucleotide sequences for site specific, in-frame integration of said nucleic acid molecule into the coding sequence of at least one host (poly)peptide of interest, wherein said tag comprises the interacting residues of the interaction partner and (b) assessing the expression level of the, *thus*, *tagged* gene.

The term "(poly)peptide" refers alternatively to peptide or to polypeptide. Peptides conventionally are covalently linked amino acids of up to 30 residues, whereas polypeptides (also referred to as "proteins") comprise 31 and more amino acid residues.

The term "biomolecule" relates, to a (poly)peptide or an RNA molecule or a ribonucleoprotein which is capable of modulating the amount of gene expression, preferably the amount of protein expression. "Modulating gene expression" means inhibiting or enhancing gene expression. An example of a biomolecule which is capable of inhibiting the amount of gene expression is the Lac repressor. By binding to its recognition site, the Lac repressor is capable of inhibiting the expression of open reading frames which are located downstream of this recognition site. Alternatively, the biomolecule can be an enhancer or activator of gene expression. An example of an enhancer capable of increasing gene expression is the AraC protein or the cAMP responsive protein (CRP). Preferably, the biomolecule is a nucleic acid binding biomolecule. More preferably, the biomolecule is a nucleic acid binding (poly)peptide selected from the group consisting of regulator of transcription, regulator of translation, recombinase, (poly)peptide involved in RNA transport or a ribozyme capable of regulating transcription or translation. Even more preferred are (poly)peptides comprising the (poly)peptide sequence of tetracycline repressor; Lac repressor; Xylose repressor; AraC protein; TetR-based T-Rex system (Yao et al., 1998, distributed by Invitrogen); erythromycin-specific repressor MphR(A) (Weber 2002); Pip (pristinamycin interacting protein, Fussenegger et al., 2000); ScbR (quorum sensing regulatory protein from *Streptomyces coelicolor,* Weber et al., 2003); TraR (quorum sensing regulatory protein of *Agrobacterium tumefaciens*); fused to the eukaryotic activation domain p65 of NFκB (Neddermann et al., 2003); chimeric proteins consisting of the: (i) Gal4 DNA-binding domain and either a full-length PhyA protein (PhyA-GBD) or the N-terminal photosensory domain of PhyB [PhyB(NT)-GBD] of *Arabidopsis thaliana* (ShimizuSato et al., 2002); (ii) steroid hormone regulated systems like the GeneSwitch regulatory system from Valentis (www.geneswitch.com), sold by Invitrogen (catalog number K1060-02) and consisting of (1) a Gal4 DNA-binding domain fused to a human progesterone receptor ligand binding domain and an NFκB-derived p65 eukaryotic transcription activation domain and (2) the inducer mifepristone; (iii) dimerizer systems from ARIAD consisting of (1) a ZFHD1 DNA-binding domain fused to FKBP 12, (2) a modified form of FRAP, termed FRB, fused to the NFκB-derived p65 activation domain and (3) rapamycin, AP22565 or AP12967 as heterodimer-forming agents, as they bind to both (1) and (2); (iv) Ecdysone-Inducible Expression System (with the Inducing Agents Ponasterone A and Muristerone A) from Invitrogen (catalog numbers K1001-01 K1003-01, K1004-01) containing (1) a modified form of the *Drosophila* ecdysone receptor (VgEcR) fused to a VP16 activation domain, (2) the mammalian homologue RXR of *ultraspiracle,* the natural binding partner of the ecdysone receptor in *Drosophila* and (3) the inducers ponasterone A and muristerone A.

The term "fragment" relates to the functional domains of a protein. These may be, for example, a nucleic acid binding domain, a multimerization domain or an effector domain. The person skilled in the art knows that such domains may be linked by appropriate linkers. Although not every combination of domains may result in a functional chimeric protein, the skilled person can easily determine whether a specific chimeric protein has biological activity. In many cases no precise domain boundaries may exist so that the functional domain may have additional N- and C-terminal amino acid residues.

Nucleic acid binding (poly)peptides are known to have at least two states: in their "off-state", nucleic acid binding (poly)peptides have a low affinity to their binding sequence on the nucleic acid molecule, whereas in their "on-state", nucleic acid binding (poly)peptides have a high affinity for their binding sequence. Accordingly, when the biomolecule is a nucleic acid binding (poly)peptide, the term "modulating the activity of a biomolecule" refers to the regulation of the nucleic acid binding activity of the biomolecule. "Modulating the activity" can in some cases include, in addition to "on-states" and "off-states", a number of intermediate states which becomes particularly evident when the biomolecule is an allosterically regulated protein, which are also comprised by the term "biomolecule" (see for example Genes, Lewin, J. Wiley & Sons, 1983, 1^{st} edition, page 222, 1^{st} col., last paragraph to 2^{nd} col. last paragraph). The term "monitoring" means observe the activity or expression level of a protein. "Monitoring" comprises direct or indirect measures. Preferred are indirect measures which rely on an effect of the biomolecule on, for example, transcription or translation of RNA. Particularly preferred are indirect measures based on the expression of a reporter protein. In these cases, monitoring means measuring the amount or activity of the reporter protein.

The term "host cell" means eukaryotic or prokaryotic cell. The cell can be the cell of a uni-cellular or multi-cellular organism, which may be pathogenic or non-pathogenic. Preferably, the host cell is selected from mammalian, insect, nematode, plant, yeast, protist cell, gram-positive or gram-negative bacteria, archaebacteria or protozoa. Preferably the cell is a cell expressing an allosterically regulated biomolecule. More preferably, the cell is a cell expressing a biomolecule which is a nucleic acid binding (poly)peptide. Said biomolecule can be expressed transiently or, more preferable, stably.

The term "transforming", is used herein synonymous with transfection and means introducing a nucleic acid molecule into a cell. Cells can be transformed by various methods known in the art, including methods such as electroporation, lipofection, viral infection, phage infection, microinjection. The term "introducing" refers to method of transfecting or transforming a host cell with a nucleic acid molecule encoding, for example, a (poly)peptide comprising an interaction partner of the biomolecule. Introduction of the construct into the host cell can be effected by calcium phosphate transfection, DEAE-dextran mediated transfection, cationic lipid-mediated transfection, electroporation, transduction, infection or by other methods. Such methods are described in many standard laboratory manuals, such as Davis et al., Basic Methods In Molecular Biology (1986) or Sambrook et al., "Molecular Cloning, A Laboratory Manual"; ISBN: 0879695765, CSH Press, Cold Spring Harbor, 2001.

The term "nucleic acid molecule" comprises DNA and RNA which may be single stranded or double stranded, circular or linear. "Nucleic acid molecules encoding an interaction partner" relates to expression constructs capable of mediating protein expression. Said nucleic acid molecule introduced into the host cell comprises an open reading frame encoding an interaction partner of said biomolecule in expressible form. "Expressible form" implies that the nucleic acid molecule may contain regulatory sequences that allow expression of the encoded (poly)peptide upon introduction of the nucleic acid molecule into the host cell.

The nucleic acid molecule introduced into the host cell may be part of an expression vector. Such vectors are usually specific for prokaryotic or eukaryotic expression. However, the nucleic acid molecule may also be part of a vector functional in prokaryotes and eukaryotes.

A typical prokaryotic expression vector usually contains a promoter element, which mediates the initiation of transcription of mRNA, the protein coding sequence, and signals required for the termination of transcription of the transcript. Transcription of DNA is dependent upon the presence of a promoter which is a DNA sequence that directs the binding of RNA polymerase and thereby promotes mRNA synthesis. The DNA sequences of eukaryotic promoters differ from those of prokaryotic promoters. Furthermore, eukaryotic promoters and accompanying genetic signals may not be recognized in or may not function in a prokaryotic system, and, further prokaryotic promoters are not recognized and do not function in eukaryotic cells. Similarly, translation of mRNA in prokaryotes depends upon the presence of the proper prokaryotic signals which differ from those of eukaryotes.. Promoters vary in their "strength" (i.e. their ability to promote transcription). In some cases it may be desirable to use strong promoters in order to obtain a high level of transcription and, hence, expression of a protein. Depending upon the host cell system utilized, any one of a number of suitable promoters may be used. For instance, when using *E*. *coli*, its bacteriophages, or plasmids, promoters such as the T7 phage promoter, *lac* promoter, *trp* promoter, *recA* promoter, ribosomal RNA promoter, the *P*_{R}. and *P*_{L} promoters of coliphage lambda and others, including but not limited, to *lac*UV5, *ompF, bla, lpp,* and the like, may be used to direct high levels of transcription of adjacent DNA segments. Additionally, a hybrid *trp-lac*UV5 (*tac*) promoter or other *E*. *coli* promoters produced by recombinant DNA or other synthetic DNA techniques may be used to provide for transcription of the inserted gene. Specific initiation signals are also required for efficient gene transcription and translation in prokaryotic cells. These transcription and translation initiation signals may vary in "strength" as measured by the quantity of gene specific messenger RNA and protein synthesized, respectively. The DNA expression vector or nucleic acid molecule encoding a (poly)peptide, which contains a promotor, may also contain any combination of various "strong" transcription and/or translation initiation signals. For instance, efficient translation in E. coli requires an SD sequence about 7-9 bases 5' to the initiation codon ("ATG") to provide a ribosome binding site. The SD sequences are complementary to the 3'-end of the 16S rRNA (ribosomal RNA) and probably promote binding of mRNA to ribosomes by duplexing with the rRNA to allow correct positioning of the ribosome. For a review on maximizing gene expression, see Roberts and Lauer, Methods in Enzymology, 68:473 (1979), which is hereby incorporated by reference. Thus, an SD-ATG combination that can be utilized by host cell ribosomes may be employed. Such combinations include but are not limited to the SD-ATG combination from the cro gene or the N gene of coliphage lambda, or from the E. coli tryptophan E, D, C, B or A genes. Additionally, any SD-ATG combination produced by recombinant DNA or other techniques involving incorporation of synthetic nucleotides may be used. Suitable expression vectors for use in practicing the present invention include, for example, vectors such as pET expression vectors (Novagen), pQE expression vectors (Qiagen), pASK expression vectors (IBA), pBAD expression vectors (Invitrogen), pWH1950 (Ettner et al., 1996), pWH1520 (MoBiTec), pWH353 with a Xyl/Tet promoter (Geissendörfer and Hillen, 1990), pLZ vectors with SPAC, Xyl and Xyl/Tet promoter systems (Zhang et al., 2000) or pNZ8008 with a *nisA* promoter (Eichenbaum et al., 1998).

A typical eukaryotic expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin, EF-1α and ubiquitin C promoters). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). Host cells that could be used include human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. Alternatively, the encoded (poly)peptides can be expressed in stable cell lines that contain a nucleic acid molecule of interest integrated into a chromosome. The co-transfection with a selectable marker such as dhfr, gpt, neomycin, hygromycin allows the identification and isolation of the transfected cells. The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy et al., *Biochem J.* 227:277-279 (1991); Bebbington et al., *Bio*/*Technology 10*:169-175 (1992)). Using these markers, the cells are grown in selective medium and the cells with the highest resistance are selected. These cell lines contain the amplified gene(s) integrated into a chromosome. Chinese hamster ovary (CHO) and NSO cells are often used for the production of proteins. The expression vectors pC1 and pC4 contain the strong promoter (LTR) of the Rous Sarcoma Virus (Cullen *et al., Molecular and Cellular Biology,* 438-447 (March, 1985)) plus a fragment of the CMV-enhancer (*Boshart et al.*, *Cell 41*:521-530 (1985)). Multiple cloning sites, e.g., with the restriction enzyme cleavage sites *Bam*HI, *Xba*l and *Asp*718, facilitate the cloning of the gene of interest. The vectors contain in addition the 3' intron, the polyadenylation and termination signal of the rat preproinsulin gene. As indicated above, the expression vectors will preferably include at least one selectable marker. Such markers include dihydrofolate reductase, Herpes simplex virus thymidine kinase, G418 or puromycin or zeocin resistance for eukaryotic cell culture and tetracycline, hygromycin, apramycin, zeocin, kanamycin or ampicillin resistance genes for culturing in *E. coli* and other bacteria. Representative examples of appropriate hosts include, but are not limited to, bacterial cells, such as *E. coli, Streptomyces* and *Salmonella typhimurium* cells; *Staphylococcus aureus*, *Streptococcus pneumoniae*, *Corynebacterium glutamicum* or *Bacillus subtilis;* fungal cells, such as yeast cells; insect cells such as *Drosophila* S2 and *Spodoptera* Sf9 cells; animal cells such as CHO, COS, 293 and Bowes melanoma cells; and plant cells. Appropriate culture mediums and conditions for the above-described host cells are known in the art.

The term "interaction partner of said biomolecule" refers to a string of amino acid residues with affinity to a (poly)peptide or aptamer, allosteric ribozyme, riboswitch or ribonucleoprotein. Said string of amino acid residues may be a peptide or a polypeptide. Preferably, the string of amino acids with affinity to the biomolecule is part of a fusion protein. The interaction partner may be present at the carboxy terminal end of the fusion protein, it can, however, also be located at the amino terminal end or at an internal position of the amino acid sequence of the protein, provided that this is not associated with a negative property such as e.g. impeding or destroying the biological activity etc. The larger subunit of the fusion protein can be a complete protein or a mutant of a protein such as e.g. a deletion mutant or substitution mutant. If the biomolecule is the Tet repressor or comprises (poly)peptide sequences of the Tet repressor, the interaction partner is preferably the polypeptide of the present invention or the polypeptide expressed by the nucleic acid molecule of the present invention or a fusion protein comprising said (poly)peptide.

This is experimentally feasible, as different sets of more than 6000 yeast strains, each expressing a distinct GST-ORF, GFP-ORF or TAP-ORF fusion, have already been generated (Ghaemmaghami et al., 2003; Huh, et al., 2003; Martzen et al., 1999).

Components of tetracycline (tc)-dependent gene regulation systems are commonly used and have been introduced into almost every eukaryotic and prokaryotic model organism to conditionally control the expression of individual target genes. Thus, transgenic organisms and cell lines containing tc-dependent regulatory systems are widely distributed among academic and industrial research groups. Typically, the expression of a gene is controlled by externally applying tc or one of its more active derivatives, doxycycline or anhydrotetracycline. Using phage display, as disclosed in the present invention, we have now identified an oligopeptide that, when expressed as a fusion with another protein like, for example, thioredoxin (Trx), can induce expression of a gene under control of TetR *in vivo.* Such a peptide has not been described for TetR or any other allosteric regulatory protein. If the reporter gene under Tet control is sensitive and can be quantified over a wide expression range, like firefly luciferase, it is possible to quantify the expression of an endogenous protein that has been tagged with the peptide. If the reporter gene expressed is green fluorescent protein, fluorescence activated cell sorting techniques can be used to separate cells that express the tagged protein from those that do not. In bacteria, for example, this system can be used as a genetic reporter system to address questions or problems in proteomics, once a collection of mutant strains has been constructed that each contain a differently tagged protein, thus, representing the entire proteome of the respective bacterium. The "ensemble" of these strains can then be subjected to different growth conditions, like heat shock, N source limitation, sporulation or the onset of stationary phase. The expression of the reporter gene in an individual bacterium will reflect the expression pattern of the respective "tagged" protein and the collection of tagged strains, thus, the "proteome" of the respective condition. The intensity of the reporter gene signal correlates to the expression level of the expressed and tagged protein.This allows the introduction of genetic methods to the field of proteomics. According to the present state of the art, this still requires protein-biochemical methods which are much more laborious and time-consuming. Tagging approaches to detect protein expression visually by fusing GFP to the C-terminus of an open reading frame (Huh et al., 2003) or to quantify protein expression by fusing a "TAP" tag to the C-terminus of an open reaging frame (Ghaemmaghami et al., 2003) allow either separation of cells expressing a tagged protein by FACS and quantification in the case of highly expressed proteins (GFP-tag) or a precise quantification of a tagged proteins' expression level (TAP-tag). Thus, quantification and visual detection requires the introduction of two independent tags per protein.

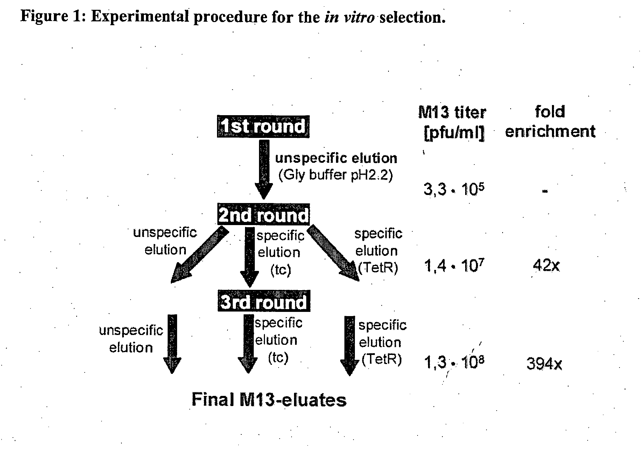

The invention can be used to facilitate proteome analysis. First, a bacterial population has to be generated that contains a set of individual bacteria that, together, constitute the proteome of the organism. To achieve this, every single bacterium in this population carries a single encoded protein that has been tagged with the interacting peptide. In *Bacillus subtilis* or *Escherichia coli,* this would correspond to about 4000 different strains. In other organisms, like *Streptomyces coelicolor,* the number of strains needed would be about 7500, in *Mycoplasma pneumoniae* only about 650. This is experimentally feasible, as a set of 6144 yeast strains, each expressing a distinct GST-ORF fusion, has already been generated (Martzen et al., 1999). Automated parallel approaches have allowed generating up to 400 different expression vectors over a three day period. For an organism with a genome about the size of yeast, this time-frame would extrapolate to a few months of work (Phizicky et al., 2003). (see figure 1)

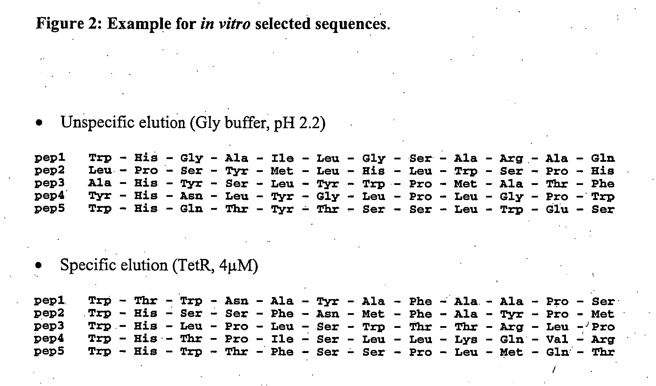

Once the set of strains has been generated, the bacterial population carrying the tagged proteome can be used to explore the bacterial proteome. The bacterial population would be grown under different conditions, e. g. rich medium, minimal medium, different growth temperatures, alternative carbon or nitrogen sources, logarithmic phase, stationary phase, exposure to different stresses like changes in the osmolarity, oxygen availability, different antibiotics or bacterial toxins, to name a few, but not necessarily all possible altered growth conditions. This could be done in an array-like setup in microtiter plates to facilitate identification of expressed protein or, more classically, on plates. Strains that express a tagged protein under the respective environmental conditions are identified by GFP fluorescence, either visually or under a fluorescence-microplate reader. The corresponding proteins are identified either by reading-out the position in the strain array or by direct sequencing of genomic DNA (ABI Prism 310 Genetic Analyzer User's Manual) using the known DNA sequence of the tag. (see figure 2)

The teaching of the present application is particularly useful in disease proteomics. As of yet, proteome analysis of pathogenic microorganisms tries to mimic *ex vivo* the environmental conditions supposed to be encountered by the microorganism in the host *[(Streptococcus mutans -* (Len et al., 2003); *Salmonella typhimurium -* (Deiwick et al., 2002); *Pseudomonas aeruginosa* - (Guina et al., 2003); *Leishmania infantum -* (El Fakhry et al., 2002); *Mycobacterium tuberculosis -* (Rosenkrands et al., 2002)]. *In vivo* analysis of pathogenicity-specific genes is mainly conducted by microarray analysis of mRNA expression (Chan et al., 2002; Eriksson et al., 2003; Okinaka et al., 2002; Staudinger et al., 2002) or analysis of bacterial strains expressing different antisense constructs (Ji et al., 2001). The invention described here can facilitate the analysis of protein expression in the pathogenic organism in an *in vivo* animal disease model. The animal model is infected using one of the methods of the present invention by a bacterial population containing several copies of the tagged proteome. The disease is allowed to develop. At an appropriate timepoint, the animal is sacrificed, the respective organs and tissues containing the pathogenic microorganism are isolated, bacterial translation is arrested by addition of a bacteristatic translation inhibitor like chloramphenicol and the tissue (if necessary) is homogenized. Bacteria are isolated by filtration and sorted by FACS (fluorescence activated cell sorting) into GFP-expressing and GFP-non-expressing populations. These are sorted into single wells and streaked to clonal populations. Due to the defined tag sequence, the individual protein that was expressed in the host organism can then be identified by direct genomic sequencing.

The teaching of the present application is also particularly useful to facilitate analysis of expression of proteins that are difficult to detect by conventional proteomics. Low-abundance proteins like transcriptional regulators or signal transducing proteins like kinases are often not detectable by standard proteome methods, even though many of these proteins are important for pathogenesis or development (Deiwick et al., 2002; Gygi et al., 2000). Induction of reporter gene expression by binding of a tagged protein to TetR leads to amplification of the initial signal by allowing multiple transcription events and subsequent multiple rounds of translation per mRNA molecule.

Moreover, the teaching of the present application is also particularly useful to allow exogenous control of endogenous riboswitch-controlled translational regulation systems (Stormo, 2003). Riboswitches have been identified in all three kingdoms (Sudarsan et al., 2003). Examples for molecules that bind to riboswitches are thiamine (Winkler et al., 2002a), S-adenosylmethionine (Epshtein et al., 2003; Winkler et al., 2003), FMN (Winkler et al., 2002b) and guanine (Mandal et al., 2003). These are all common metabolites and their intracellular concentration cannot be easily manipulated. Inducible expression of peptides that recognize these RNA elements and can flip the switch would allow external control of central metabolic pathways without having to interfere with the natural genetic situation through the introduction of foreign control elements (repressors, activators) in classical transcription control.

In a preferred embodiment of the present invention's method, the activity or degree of modulation of the activity of the biomolecule is measured via a readout system. The readout system can be based on the detection of a transcription product, a translation product or on the activity of translated (poly)peptide. Readout system can be any detectable product, the expression level of which is modulated by the action of the biomolecule. Readout may be a signal of a specific wavelength such as those generated by GFP or luciferase or the like. Also comprised by the present invention are enzyme based readout systems such as the CAT system. The readout system is controlled by a binding site for the regulatory biomolecule. For example, in the case of a biomolecule with repressing activity, the binding site controlling the readout system is bound by said "biomolecule with repressing activity" and thereby expression from said readout system is blocked. Upon binding by the interaction partner disclosed in the present invention, the biomolecule is released and expression from the readout system is achieved.

In a more preferred embodiment of the present invention's method, (a) the readout system is provided by a nucleic acid molecule encoding a reporter protein; (b) the biomolecule is (i) a nucleic acid binding (poly)peptide selected from the group consisting of regulator of transcription, regulator of translation, recombinase, (poly)peptide involved RNA transport or (i) an aptamer, allosteric ribozyme or riboswitch and (c) the nucleic acid binding biomolecule is allosterically regulated.Aptamer, allosteric ribozyme or riboswitch are RNA molecules capable of performing a conformational switch after ligand binding. Peptide binding to RNA has been demonstrated by Rev-RRE (Gosser et al., 2001; Harada et al., 1996; Harada et al., 1999; Zhang et al., 2001) or Tar-TAT (Calnan et al., 1991; Weeks et al., 1990), indicating that selection of peptides binding to a ligand binding site is a feasible approach. The principle of allosteric ribozymes is outlined in (Soukup and Breaker, 1999) and well known examples are hammerhead ribozymes dependent on the presence of ATP (Tang and Breaker, 1997), theophylline (Soukup et al., 2000) or cGMP/cAMP (Koizumi et al., 1999) for activity. Several examples of riboswitches have been described in the literature. The best described is the vitamin B1-dependent *thiM* switch from *Escherichia coli* (Winkler et al., 2002a). Two examples of artificially generated ligand-controlled translational switches are described in (Suess et al., 2003) and (Werstuck and Green, 1998). The present invention particularly refers to the following RNA-binding (poly)peptides and their recognition sequences which may be used in accordance with the teaching of the present invention and in any of the methods disclosed in the present invention:REV binding peptide: clone 3-AAAAGRRARRRRRRRRQSCRRKMTRD (Tan and Frankel, 1998); RRE site: 5'-UGGGCGCAGCGUCAAUGACGCUGACGGUACA-3' (Peterson and Feigon, 1996); TAT peptide fragment: Tfr38-RKKRRQRRRPPQGSQTHQVSLSKQPTSQPRGDPTGPKE (Weeks et al., 1990); TAR site: 5'-GGUCUCUCUGGUUAGACCAGAUCUGAGCCUGGGAGCUCUCUGGCUAACUAG AGAACCC-3' (Weeks et al., 1990); ATP-sensitive allosteric ribozyme: (i) ribozyme strand: 5'-GGGCGACCCUGAUGAGUUGGGAAGAAACUGUGGCACUUCGGUGCCAGCAAC GAAACGGU-3'; (ii) substrate strand: 5'-GCCGUAGGUUGCCC-3' (Tang and Breaker, 1998); theophylline-sensitive allosteric ribozyme: cm⁺theo5: 5'-GGGCGACCCUGAUGAGCCUGGAUACCAGCCGAAAGGCCCUUGGCAGUUAGA CGAAACGGUGAAAGCCGUAGGUUGCCC-3' (Soukup et al., 2000); cGMP-sensitive allosteric ribozyme: cGMP 1: 5'-GGGCGACCCUGAUGAGCCUUGCGAUGCAAAAAGGUGCUGACGACACAUCGA AACGGUGAAAGCCGUAGGUUGCCC-3' (Koizumi et al., 1999); *Escherichia coli thiM* riboswitch: 5'-GGAACCAAACGACUCGGGGUGCCCUUCUGCGUGAAGGCUGAGAAAUACCCG UAUCACCUGAUCUGGAUAAUGCCAGCGUAGGGAAGUCACGGACCACCAGGU CAUUGCUUCUUCACGUUAUGGCAGGAGCAAACUAUGCAAGUCGACCUGCUG GAUCCAGCGCAA-3' (Winkler et al., 2002a); tetracycline-dependent translational switch: cb32 5'-CUUAAGGCCUGUACUGCUGCUUAAGGCCUAAAACAUACCAGAUCGCCACCC GCGCUUUAAUCUGGAGAGGUGAAGAAUACGACCACCUAGGCCAAAAUGGCU AGC-3' (Suess et al., 2003); Hoechst33258-specific translational switch: H19 5'-GGUGAUCAGAUUCUGAUCCAACAGGUUAUGUAGUCUCCUACCUCUGCGCCU GAAGCUUGGAUCCGUCGC-3' (Werstuck and Green, 1998);

The methods disclosed in the present invention allow to develop peptides or polypeptides capable of interacting with the above-mentioned biomolecules. In accordance with the present invention, it is required that the interacting (poly)peptides not only bind to the biomolecules but also modulate their activity, preferably their nucleic acid binding affinity. For example by using phage display or antibody libraries, new interaction partners of the Tet repressor might be identified which are capable of reducing the affinity of the Tet repressor for its recognition sequence. These newly identified sequences can either be expressed on their own or as part of a fusion protein and, thus, regulate the expression of the reporter protein located downstream of the recognition sequence of the Tet repressor.

Although only specific examples for interaction partners of Tet repressor are disclosed in the present invention, the skilled person can, based on the teaching of the present invention, identify modulators of any other nucleic acid binding protein. Generally, the interaction partner is a biological macromolecule. Preferably, new interaction partners are identified by using phage display or by screening antibody libraries. Alternatively, interaction partners may be identified by ribozyme display, mRNA display, cell-surface display and/or lipocalin-based libraries. Peptide libraries based on these scaffolds (lipocalins, cell surface display, mRNA display) have been constructed, published and successfully used to select target-specific peptides.

Phage display and combinatorial methods for generating interaction partners are known in the art (as described in, e.g., Ladner et al. U.S. Pat. No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Bio/Technology 9:1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; and Barbas et al. (1991) PNAS 88:7978-7982, the contents of all of which are incorporated by reference herein).

The basis for most phage display applications is the filamentous phage M13 from *Escherichia coli.* A surface protein, gplll which is present in five copies per phage and important for infection of the bacteria can tolerate N-terminal insertions to a certain degree. Since the N-terminus of this protein is solvent-exposed, the insertions are presented on the surface of the phage (Kay et al., 2001). The commercially available phage bank Ph.D.-12™ from New England Biolabs is preferably used to select for interaction partners. Alternatively, : Ph.D.-7™, Ph.D.-C7C™ (New England Biolabs), FliTrx Random Peptide Display Library (Invitrogen), Ready-To-Use Phage Display cDNA Libraries (Spring Bioscience) or pSKAN Phagemid Display System (MoBiTec) may be used. Phage bank Ph.D.-12™ contains ~10⁹ different dodecapeptides fused via a flexible linker of four amino acids to the N-terminus of the gpIII protein. The *in vitro* selection may be performed by coating polystyrene tubes (NUNC Maxisorb) with purified biomolecule. Generally, the tubes are then incubated with the pool of M13 phages, washed several times and phages are eluted either specifically by addition of biomolecule or unspecifically by lowering the pH value. Individual M13 clones are usually picked and sequenced after three selection rounds.

Binding of individual phage clones to the biomolecule may be determined by using an ELISA (Enzyme Linked Immunosorbent Assay). The phages are amplified, precipitated and resuspended in a small volume. Subsequently, 96-well microtiterplates may be coated with the biomoleucle, then blocked with a blocking reagent such as Bovine Serum Albumin, followed by incubation with increasing amounts of M13 phages from different isolates, several washes with buffer and, finally, incubated with a phage-specific monoclonal antibody covalently coupled to horseradish peroxidase. Addition of ABTS (2', 2'-Azino-bis(ethylbenzthiazolin-6-sulfonic acid) as substrate permits the spectophotometric detection of phage-binding to TetR. The degree of absorption thereby serves as a quantitative indicator of phages bound to the target protein (Kay et al., 2001).

Repressor-inducing interaction partners may be isolated as discussed below. Since small oligopeptides are rapidly degraded intracellulary by proteases, the peptide-encoding sequences may be cloned as C-terminal fusions to the *Escherichia coli* protein thioredoxin, an established carrier protein for peptides (Park and Raines, 2000). The thioredoxin fusion proteins can be expressed, for example, by using a plasmid containing a tac promoter under control of Lac repressor (Ettner et al., 1996). The biomolecule of interest may be expressed constitutively at a low level. Preferably, in particular if the regulatory biomolecule is the Tet repressor, the indicator strain is *E*. *coli* DH5α(λ*tet*50) containing the phage λ*tet*50 (Smith and Bertrand, 1988) integrated in single copy into the *E*. *coli* genome. This phage contains a *tet*A-*lac*Z transcriptional fusion. Expression of β-galactosidase is, thus, regulated by TetR that binds to *tet*O sequences located within the promoter. The pool of potential interaction partners is screened, for example, by plating transformed colonies on MacConkey agar containing IPTG. When using the above-indicated expression vector under suitable conditions, an inducing interaction partner of the biomolecule, in this case of the Tet repressor, will lead to the expression of β-galactosidase, resulting in an acidification of the medium surrounding the colony which can be detected by its yellow color.

Preferably, individual candidates identified by sequencing are cloned as fusion proteins, transformed into suitable reporter strains. For example, if the repressor is the Tet repressor, the bacterial strain DH5α(*λtet*50) may be used and the respective β-gaiactosidase activity is subsequently determined. For example, if the repressor is the Tet repressor, the following controls might also be included in the measurements: To define the regulatory window, both the repressed state (0% - TetR binds to *tet*O) and the fully induced state in the presence of tc (100% - TetR dissociates from *tet*O) may be determined. To exclude that the remainder of the fusion protein, i.e. the carrier protein, interacts unspecifically, i.e. by amino acid residues of the carrier protein and not by amino acid residues of the interaction partner, a plasmid expressing the carrier protein without a peptide fusion may also be assayed in the presence and absence of IPTG. The β-galactosidase activities of the individual candidates cloned can also determined in the presence and absence of IPTG.

Interaction sites between the interaction partner and the biomolecule may be determined by epitope mapping. Particularly preferred is the isolation of the interaction site by *in vivo* epitope mapping, taking advantage of the observation that TetR(B/D) is not induced by the peptide. Chimeric repressor molecules may be constructed in which tetR(B) sequences are exchanged to different extents by the corresponding sequences from tetR(D) (Schnappinger et al., 1998). *In vivo* inducibility is determined by TrxA-pepBs1. In the case of Tet repressor, the results of inducibility profiles show that interactions between repressor and peptide are confined to the region from helix α8 to residue 182 in helix α10 (see table 1). The loop connecting the helices α8 and α9 also appears to be important, as a chimera containing *tetR*(D) sequences at residues 153-167 is not inducible by TrxA-pepBs1. The loop between helices α9 and α10 also seems to be important, as chimeras containing tetR(D) sequences between residues 179-184 and 180-184 are not inducible by TrxA-pepBs1.

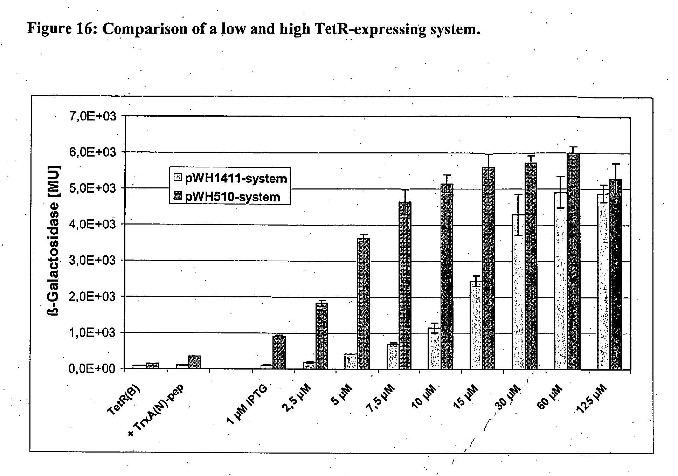

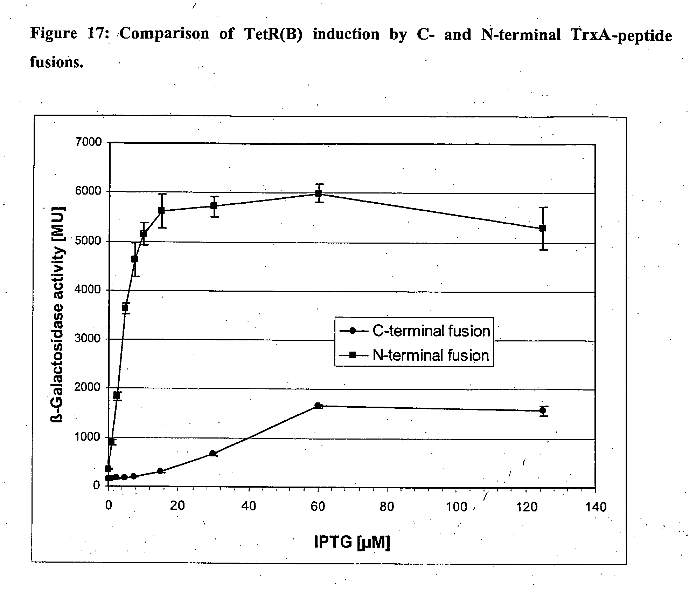

In particular cases it may be desirable to increase or decrease the strength of the interaction between the biomolecule and the interacting peptide or polypeptide. In these cases, the interaction can be modified by
(i) altering the fusion point of the peptide with the target protein. For thioredoxin, an N-terminal fusion induces TetR more sensitively than a C-terminal one (Figure17),
(ii) by altering the expression level of TetR. TetR is expressed constitutively to a higher level from a pWH1411 - derivative than from a pWH510-derivative (Wissmann et al., 1991). Consequently, higher amounts of N-terminally peptide-tagged thioredoxin are needed for full induction of β-galactosidase activity (Figure 16) and/or by
(iii) selectively exchanging, adding or deleting amino acid residues within the interacting (poly)peptide or the biomolecule. Optimal interaction may be achieved by using a rational design or by randomizing particular subunits of the (poly)peptide or biomolecule. Rational design of interaction partners may first require to obtain structural information of the interacting (poly)peptide and the biomolecule or to use available structural information. For example, by using the three-dimensional information available for the Lac repressor in a method of computer modeling, new peptide sequences can be identified, capable of binding to and inducing a conformational change in the Lac repressor which will then modify the nucleic acid binding affinity of the Lac repressor. On the other hand, known modulators of the biomolecules might be used in any of the methods of the present invention. Examples of proteins as cofactor of a regulator include phosphorylated Hpr and CcpA from *Bacilli* (Aung-Hilbrich et al., 2002), TnrA and feedback-inhibited glutamine synthetase from *Bacillus subtilis* (Wray et al., 2001), Mlc and dephosphorylated PtsG (Lee et al., 2000), or MalT and MalY (Schreiber et al., 2000) from *Escherichia coli*. Moreover, known interaction partners of regulatory biomolecules may be expressed, for example in a phage display library, wherein portions of their sequence are randomized. This will allow to isolate new interaction partners with modified properties, either binding more tightly or less tightly to the biomolecule.

In a preferred embodiment of the present invention's method, the transformed nucleic acid molecule encoding the interaction partner of the regulatory biomolecule is a nucleic acid molecule comprising: (a) a nucleic acid sequence encoding a peptide tag or a tagged (poly)peptide, operatively linked to expression control sequences; or (b) a nucleic acid sequence encoding a tagged (poly)peptide or a peptide tag, a selectable marker gene and additional nucleotide sequences for site specific, in-frame integration of said nucleic acid molecule into the coding sequence of at least one host (poly)peptide of interest. Constructs containing a nucleic acid sequence encoding a tagged (poly)peptide or a peptide tag, a selectable marker gene and additional nucleotide sequences for site specific, in-frame integration of said nucleic acid molecule into the coding sequence of at least one host (poly)peptide of interest are particularly useful for generating host cells expressing tagged host proteins. Such constructs allow in-frame integration of tag encoding sequences into chromosomal sequences encoding, for example, a host protein. A DNA module that begins with the interaction tag-encoding sequence and includes a both excisable and selectable marker (present on plasmids like pKD3, pKD4, pKD13 (Datsenko and Wanner, 2000)) may be amplified by a standard polymerase chain reaction with primers that carry extensions homologous to the C-terminal end of the targeted gene and to a region downstream of it in the host genome. Transformation of a strain expressing bacteriophage λ red functions [encoded, for example, on a plasmid like pKD20 (Datsenko and Wanner, 2000)] yields recombinants carrying the targeted gene fused to the interaction tag-encoding sequence [(Uzzau et al., 2001); a modification of a method originally described by Datsenko and Wanner (2000)]. The selectable marker may be, but is not limited to, an expression cassette conferring resistance against an antibiotic like ampicillin, apramyin, chloramphenicol, erythromycin, hygromycin, kanamycin, tetracycline, or zeozin. It is flanked by recognition sites for site-specific recombinases, like FRT/Flp [expressed from a plasmid like pCP20 (Cherepanov and Wackernagel, 1995)] or loxP/Cre. The PCR primers are between 56 to 60 nucleotides long, (i) anneal to constant regions of 20 to 21 nucleotides of the amplification template and (ii) carry extensions of 36 to 40 nucleotides that are identical in sequence to the last portion of the gene to be targeted (downstream primer) and to a region downstream of the gene (upstream primer). The PCR fragments are purified and introduced into the strain of interest and selected for recombinants carrying the amplified sequence in the genome. As a result of this targeted insertion, the host protein contains an N-terminal, C-terminal or internal amino acid sequence capable of interacting with the regulatory biomolecule. Upon expression of the host protein, the biomolecule will interact with the tagged host protein. In cases when the biomolecule is a nucleic acid binding (poly)peptide, this interaction results in a modulation of the biomolecules' affinity to its recognition sequence. For example a repressor might be switched into its inactive state and thereby allow transcription of the gene controlled by the repressor.

As outlined above, the interaction partner of the biomolecule may be a peptide tag or a tagged (poly)peptide. A tagged (poly)peptide is a fusion protein containing amino acid residues capable of interacting with the biomolecule. Preferred fusion peptides are those encoded by the nucleic acid molecules of the present invention, in particular when the biomolecule is the Tet repressor or comprises a fragment of the Tet repressor. The interaction partner may be located at N- or C-terminal position of the fusion peptide or may be located at an internal position, provided that the activity of the biomolecule is not affected by the presence of the interaction partner. In some cases, it may be desirable to add the interaction partner externally, i.e. by adding, for example, the peptide or polypeptide to the culture medium of the cell. In these cases the interaction partner also may be a peptide attached to a carrier polypeptide. Preferably, said attachment is a covalent attachment, for example achieved by crosslinking the interacting peptide with the carrier polyprotein. The conditions and reagents required for crosslinking are known to the person skilled in the art. A comprehensive product guide and reagents for crosslinking are, for example, obtainable from Pierce Biotechnology, Inc., P.O. Box 117, Rockford, IL, 61105, USA.

In another preferred embodiment of the present invention, the nucleic acid binding (poly)peptide comprises the (poly)peptide sequence of (a) Tet repressor; (b) lac repressor; (c) Xylose repressor; (d) AraC protein; (e) TetR-based T-Rex system; (f) erythromycin-specific repressor MphR(A); (g) Pip (pristinamycin interacting protein); (h) ScbR from *Streptomyces coelicolor*; (i) TraR of *Agrobacterium tumefaciens*, fused to the eukaryotic activation domain p65 of NFκB; (j) chimeric proteins consisting of the: (i) Gal4 DNA-binding domain and either a full-length PhyA protein (PhyA-GBD) or the N-terminal photosensory domain of PhyB [PhyB(NT)-GBD] of *Arabidopsis thaliana*; (ii) steroid hormone regulated systems like the GeneSwitch regulatory system from Valentis (www.geneswitch.com), sold by Invitrogen (catalog number K1060-02) and consisting of (1) a Gal4 DNA-binding domain fused to a human progesterone receptor ligand binding domain and an NFκB-derived p65 eukaryotic transcription activation domain and (2) the inducer mifepristone; (iii) dimerizer systems from ARIAD consisting of (1) a ZFHD1 DNA-binding domain fused to FKBP 12, (2) a modified form of FRAP, termed FRB, fused to the NFκB-derived p65 activation domain and (3) rapamycin, AP22565 or AP12967 as heterodimer-forming agents, as they bind to both (1) and (2); (iv) Ecdysone-Inducible Expression System (with the Inducing Agents Ponasterone A and Muristerone A) from Invitrogen (catalog numbers K1001-01 K1003-01, K1004-01) containing (1) a modified form of the *Drosophila* ecdysone receptor (VgEcR) fused to a VP16 activation domain, (2) the mammalian homologue RXR of *ultraspiracle,* the natural binding partner of the ecdysone receptor in *Drosophila* and (3) the inducers ponasterone A and muristerone A. Particular examples of (poly)peptides which may be used in accordance with the teaching of the present invention are the following (poly)peptides: (a) **Tet repressor:** GenBank accession number: X00694 MSRLDKSKVINSALELLNEVGIEGLTTRKLAQKLGVEQPTLYWHVKNKRALLDALAI EMLDRHHTHFCPLEGESWQDFLRNNAKSFRCALLSHRDGAKVHLGTRPTEKQYE TLENQLAFLCQQGFSLENALYALSAVGHFTLGCVLEDQEHQVAKEERETPTTDSM PPLLRQAIELFDHQGAEPAFLFGLELIICGLEKQLKCESGS; (a-1) **tTA** (TetR-VP16) (Gossen and Bujard, 1992):
MSRLDKSKVINSALELLNEVGIEGLTTRKLAQKLGVEQPTLYWHVKNKRALLDALAI EMLDRHHTHFCPLEGESWQDFLRNNAKSFRCALLSHRDGAKVHLGTRPTEKQYE TLENQLAFLCQQGFSLENALYALSAVGHFTLGCVLEDQEHQVAKEERETPTTDSM PPLLRQAIELFDHQGAEPAFLFGLELIICGLEKQLKCESGSAYSRARTKNNYGSTIE GLLDLPDDDAPEEAGLAAPRLSFLPAGHTRRLSTAPPTDVSLGDELHLDGEDVAM AHADALDDFDLDMLGDGDSPGPGFTPHDSAPYGALDMADFEFEQMFTDALGIDE YGG; (a-2) **tTA2** (TetR-FFF) (Baron et al., 1997):
MSRLDKSKVINSALELLNEVGIEGLTTRKLAQKLGVEQPTLYWHVKNKRALLDALAI EMLDRHHTHFCPLEGESWQDFLRNNAKSFRCALLSHRDGAKVHLGTRPTEKQYE TLENQLAFLCQQGFSLENALYALSAVGHFTLGCVLEDQEHQVAKEERETPTTDSM PPLLRQAIELFDHQGAEPAFLFGLELI ICGLEKQLKCESGGPADALDDFDLDMLPAD ALDDFDLDMLPADALDDFDLDMLPG; (a-3) **tTA-p65** (TetR-p65) (Urlinger et al., 2000):
MSRLDKSKVINSALELLNEVGIEGLTTRKLAQKLGVEQPTLYWHVKNKRALLDALAI EMLDRHHTHFCPLEGESWQDFLRNKAKSFRCALLSHRDGAKVHLGTRPTEKQYE TLENQLAFLCQQGFSLENALYALSAVGHFTLGCVLEDQEHQVAKEERETPTTDSM PPLLRQAIELFDHQGAEPAFLFGLELIICGLEKQLKCESGSSEFQYLPDTDDRHRIE EKRKRTYETFKSIMKKSPFSGPTDPRPPPRRIAVPSRSSASVPKPAPQPYPFTSSL STINYDEFPTMVFPSGQISbASALAPAPPQVLPQAPAPAPAPAMVSALAQAPAPVP VLAPGPPQAVAPPAPKPTQAGEGTLSEALLQLQFDDEDLGALLGNSTDPAVFTDL ASVDNSEFQQLLNQGIPVAPHTTEPMLMEYPEAITRLVTGAQRPPDPAPAPLGAP GLPNGLLSGDEDFSSIADMDFSALLSQISS; (b) **Lac repressor:** GenBank accession number: J01636:
MKPVTLYDVAEYAGVSYQTVSRWNQASHVSAKTREKVEAAMAELNYIPNRVAQQ LAGKQSLLIGVATSSLALHAPSQIVAAIKSRADQLGASVVVSMVERSGVEACKAAV HNLLAQRVSGLIINYPLDDQDAIAVEi4ACTNVPALFLDVSDQTPINSIIFSHEDGTRL GVEHLVALGHQQIALLAGPLSSVSARLRLAGWHKYLTRNQIQPIAEREGDWSAMS GFQQTMQMLNEG IVPTAMLVANDQMALGAMRAITESGLRVGADISWGYDDTEDS SCYIPPSTTIKQDFRLLGQTSVDRLLQLSQGQAVKGNQLLPVSLVKRKTTLAPNTQ TASPRALADSLMQLARQVSRLESGQ; (c) **Xylose repressor:** GenBank accession number: NC000964:
MTGLNKSTVSSQVNTLMKESMVFEIGQGQSSGGRRPVMLVFNKKAGYSVGIDVG VDYINGILTDLEGTIVLDQYRHLESNSPEITKDILIDMIHHFITQMPQSPYGFIGIGICV PGLIDKDQKIVFTPNSNWRDIDLKSSIQEKYNVSVFIENEANAGAYGEKLFGAAKNH DNIIYVSISTGIGIGVIINNHLYRGVSGFSGEMGHMTIDFNGPKCSCGNRGCWELYA SEKALLKSLQTKEKKLSYQDIINLAHLNDIGTLNALQNFGFYLGIGLTNILNTFNPQAV ILRNSIIESHPMVLNSMRSEVSSRVYSQLGNSYELLPSSLGQNAPALGMSSIVIDHF LDMITM;
(d) **AraC protein:** GenBank accession number: J01641 MAEAQNDPLLPGYSFNAHLVAGLTPIEANGYLDFFIDRPLGMKGYILNLTIRGQGW . KNQGREFVCRPGDILLFPPGEIHHYGRHPEAREWYHQWVYFRPRAYWHEWLNW PSIFANTGFFRPDEAHQPHFSDLFGQIINAGQGEGRYSELLAINLLEQLLLRRMEAI NESLHPPMDNRVREACQYISDHLADSNFDIASVAQHVCLSPSRLSHLFRQQLGISV LSWREDQRISQAKLLLSTTRMPIATVGRNVGFDDQLYFSRVFKKCTGASPSEFRA GCEEKVNDVAVKLS; (e) **TetR-based T-Rex system**: the TetR sequence is identical to that in (a);
(f) **repressor protein MphR(A):** GenBank accession number: AB038042: MPRPKLKSDDEVLEAATWLKRCGPIEFTLSGVAKEVGLSRAALIQRFTNRDTLLVR MMERGVEQVRHYLNAIPIGAGPQGLWEFLQVLVRSMNTRNDFSVNYLISWYELQV PELRTLAIQRNRAVVEGIRKRLPPGAPAAAELLLHSVIAGATMQWAVDPDGELADH VLAQIAAILCLMFPEHDDFQLLQAHA; (g) ***Streptomyces coelicolor*** **transcriptional regulator Pip:** GenBank accession number AF193856 MMSRGEVRMAKAGREGPRDSVWLSGEGRRGGRRGRQPSGLDRDRITGVTVRLL DTEGLTGFSMHRLAAELNVTAMSVYWYVDTKDQLLELALDAVFGELRHPDPDAGL DWREELRALARENRALLVRHPWSSRLVGTYLNIGPHSLAFSRAVQNVVRRSGLPA HRLTGAISAVFQFVYGYGTIEGRFLARVADTGLSPEEYFQDSMTAVTEVPDTAGVI EDAQDIMAARGGDTVAEMLDRDFEFALDLLVAGIDAMVEQA; (h) ***Streptomyces coelicolor*** **transcriptional regulator ScbR:** GenBank accession number AJ007731:
   MAKQDRAIRTRQTILDAAAQVFEKQGYQAATITEILKVAGVTKGALYFHFQSKEELA LGVFDAQEPPQAVPEQPLRLQELIDMGMLFCHRLRTNVVARAGVRLSMDQQAHG LDRRGPFRRWHETLLKLLNQAKENGELLPHVVTTDSADLYVGTFAGIQVVSQTVS DYQDLEHRYALLQKHILPAIAVPSVLAALDLSEERGARLAAELAPTGKD;(i) **TraR-p65** fusion (Neddermann et al., 2003):
   MEFQYLPDTDDRHRIEEKRKRTYETFKSIMKKSPFSGPTDPRPPPRRIAVPSRSSA SVPKPAPQPYPFTSSLSTINYDEFPTMVFPSGQISQASALAPAPPQVLPQAPAPAP APAMVSALAQAPAPVPVLAPGPPQAVAPPAPKPTQAGEGTLSEALLQLQFDDEDL GALLGNSTDPAVFTDLASVDNSEFQQLLNQGIPVAPHTTEPMLMEYPEAITRLVTG AQRPPDPAPAPLGAPGLPNGLLSGDEDFSSIADMDFSALLSQISSGSARGVPKKK RKVGIQEGISAASRSMQHWLDKLTDLAAIEGDECILKTGLADIADHFGFTGYAYLHI QHRHITAVTNYHRQWQSTYFDKKFEALDPVVKRARSRKHIFTWSGEHERPTLSKD ERAFYDHASDFGIRSGITIPIKTANGFMSMFTMASDKPVIDLDREIDAVAAAATIGQI HARISFLRTTPTAEDAACVDPKEATYLRWIAVGKTMEEIADVEGVKYNSVRVKLRE RMKRFDVRSKAHLTALAIRRKLI; (j-i) The skilled person knows that almost any fusion of the Phy sequences to a Gal4-DBD (aa's 1-63, 1-95, 1-141) would be active. (j-ii) **Gal4-hpr-p65** from pSwitch (Invitrogen: Geneswitch_man.pdf):
   MDSQQPDLKLLSSIEQACDICRLKKLKCSKEKPKCAKCLKNNWECRYSPKTKRSPL TRAHLTEVESRLERLEQLFLLIFPREDLDMILKMDSLQDIKALLEFPGVDQKKFNKV RVVRALDAVALPQPVGVPNESQALSQRFTFSPGQDIQLIPPLINLLMSIEPDVIYAG HDNTKPDTSSSLLTSLNQLGERQLLSVVKWSKSLPGFRNLHIDDQITLIQYSWMSL MVFGLGWRSYKHVSGQMLYFAPDLILNEQRMKESSFYSLCLTMWQIPQEFVKLQV SQEEFLCMKVLLLLNTIPLEGLRSQTQFEEMRSSYIRELIKAIGLRQKGVVSSSQRF YQLTKLLDNLHDLVKQLHLYCLNTFIQSRALSVEFPE.MMSEVIAGSTPMEFQYLPDT DDRHRIEEKRKRTYETFKSIMKKSPFSGPTDPRPPPRRIAVPSRSSASVPKPAPQP YPFTSSLSTINYDEFPTMVFPSGQISQASALAPAPPQVLPQAPAPAPAPAMVSALA QAPAPVPVLAPGPPQAVAPPAPKPTQAGEGTLSEALLQLQFDDEDLGALLGNSTD PAVFTDLASVDNSEFQQLLNQGIPVAPHTTEPMLMEYPEAITRLVTGAQRPPDPAP APLGAPGLPNGLLSGDEDFSSIADMDFSALLSQISS; (j-iii-1) **ZHFD1-FKBP fusion** (www.ariad.com/regulationkits)
   MDYPAAKRVKLDSRERPYACPVESCDRRFSRSDELTRHIRIHTGQKPFQCRICMR NFSRSDHLTTHIRTHTGGGRRRKKRTSIETNIRVALEKSFLENQKPTSEEITMIADQL NMEKEVIRVWFCNRRQKEKRINTRGVQVETISPGDGRTFPKRGQTCVVHYTGMLE DGKKFDSSRDRNKPFKFMLGKQEVIRGWEEGVAQMSVGQRAKLTISPDYAYGAT GHPGIIPPHATLVFDVELLKLEVEGVQVETISPGDGRTFPKRGQTCVVHYTGMLED GKKFDSSRDRNKPFKFMLGKQEVIRGWEEGVAQMSVGQRAKLTISPDYAYGATG HPGIIPPHATLVFDVELLKLETRGVQVETISPGDGRTFPKRGQTCVVHYTGMLEDG KKFDSSRDRNKPFKFMLGKQEVIRGWEEGVAQMSVGQRAKLTISPDYAYGATGH PGIIPPHATLVFDVELLKLETSY; (j-iii-2) **FRB-p65 fusion** (www.ariad.com/regulationkits):
   MDYPAAKRVKLDSRILWHEMWHEGLEEASRLYFGERNVKGMFEVLEPLHAMMER GPQTLKETSFNQAYGRDLMEAQEWCRKYMKSGNVKDLLQAWDLYYHVFRRISKT RDEFPTMVFPSGQISQASALAPAPPQVLPQAPAPAPAPAIVIVSALAQAPAPVPVLA PGPPQAVAPPAPKPTQAGEGTLSEALLQLQFDDEDLGALLGNSTDPAVFTDLASV DNSEFQQLLNQGIPVAPHTTEPMLMEYPEAITRLVTGAQRPPDPAPAPLGAPGLP.N GLLSGDEDFSSIADMDFSALLSQISSTSY; (j-iv-1) **VgEcR** from pVgRXR (http://www.invitrogen.com/content/sfs/vectors/pvgrxr.pdf):
   MAPPTDVSLGDELHLDGEDVAMAHADALDDFDLDMLGDGDSPGPGFTPHDSAPY GALDMADFEFEQMFTDALGIDEYGGKLLGTSRRISNSISSGRDDLSPSSSLNGYSA NESCDAKKSKKGPAPRVQEELCLVCGDRASGYHYNALTCGSCKVFFRRSVTKSA VYCCKFGRACEMDMYMRRKCQECRLKKCLAVGMRPECVVPENQCAMKRREEKA QKEKDKMTTSPSSQHGGNGSLASGGGQDFVKKEILDLMTCEPPQHATIPLLPDEIL AKCQARNIPSLTYNQLAVIYKLIWYQDGYEQPSEEDLRRIMSQPDENESQTDVSFR HITEITILTVQLIVEFAKGLPAFTKIPQEDQITLLKACSSEVMMLRMARRYDHSSDSIF FANNRSYTRDSYKMAGMADNIEDLLHFCRQMFSMKVDNVEYALLTAIVIFSDRPGL EKAQLVEAIQSYYIDTLRIYILNRHCGDSMSLVFYAKLLSILTELRTLGNQNAEMCFS LKLKNRKLPKFLEEIWDVHAIPPSVQSHLQITQEENERLERAERMRASVGGAITAGI DCDSASTSAAAAAAQHQPQPQPQPQPSSLTQNDSQHQTQPQLQPQLPPQLQGQ LQPQLQPQLQTQLQPQIQPQPQLLPVSAPVPASVTAPGSLSAVSTSSEYMGGSAA IGPITPATTSSITAAVTASSTTSAVPMGNGVGVGVGVGGNVSMYANAQTAMALMG VALHSHQEQLIGGVAVKSEHSTTA; (j-iv-2) **RXR** from pVgRXR (http://www.invitrogen.com/content/sfs/vectors/pvgrxr.pdf):
   MDTKHFLPLDFSTQVNSSLTSPTGRGSMAAPSLHPSLGPGIGSPGQLHSPISTLSS PINGMGPPFSVISSPMGPHSMSVPTTPTLGFSTGSPQLSSPMNPVSS'SEDIKPPLG LNGVLKVPAHPSGNMASFTKHICAICGDRSSGKHYGVYSCEGCKGFFKRTVRKDL TYTCRDNKDCLIDKRQRNRCQYCRYQMCLAMGMKREAVQEERQRGKDRNENEV ESTSSANEDVPVERILEAELAVEPKTETYVEANVGLNPSSPNDPVTNICQAADKQL FTLVEWAKRIPHFSELPLDDQVILLRAGWNELLIASFSHRSIAVKDGILLATGLHVHR NSAHSAGVGAIFDRVLTELVSKMRDMQMDKTELGCLRAIVLFNPDSKGLSNPAEV EALREKVYASLEAYCKHKYPEQPGRFAKLLLRLPALRSIGLKCLEHLFFFKLIGDTPI DTFLMEMLEAPHQMT.

Particularly preferred are active fragments of said (poly)peptides.

In yet another preferred embodiment of the present invention, the reporter protein of the readout system is ß-galactosidase, CAT, ß-glucuronidase; ß-xylosidase; XylE (catechol dioxygenase); TreA (trehalase); GFP and variants CFP, YFP, EGFP, GFP+ (Scholz et al., 2000) of GFP; bacterial luciferase (*luxAB*); *Photinus* luciferase; *Renilla* luciferase; coral-derived photoproteins including DSRed, HcRed, AmCyan, ZsGreen, ZsYellow, AsRed; alkaline phosphatase or secreted alkaline phosphatase.

In a preferred embodiment of the present invention's method, the reporter protein is a protein that confers resistance to an antibiotic. In this case, it is preferred that the cells be cultivated in the presence of an antibiotic so that only clones expressing the reporter protein are capable of propagating. Generally, the protein can mediate resistance to an antibiotic such as Ampicillin, Kanamycin, Chloramphenicol, Tetracycline, Hygromycin, Neomycin or Methotrexate. Further examples of antibiotics are Penicillins: Ampicillin HCl, Ampicillin Na, Amoxycillin Na, Carbenicillin disodium, Penicillin G, Cephalosporins, Cefotaxim Na, Cefalexin HCl, Vancomycin, Cycloserine. Other examples include bacteriostatic inhibitors such as: Chloramphenicol, Erythromycin, Lincomycin, Tetracycline, Spectinomycin sulfate, Clindamycin HCl, Chlortetracycline HCl. Additional examples are proteins that allow selection with bactericidal inhibitors such as those affecting protein synthesis irreversibly causing cell death. Aminoglycosides can be inactivated by enzymes such as NPT II which phosphorylates 3'-OH present on kanamycin, thus inactivating this antibiotic. Some aminoglycoside modifying enzymes acetylate the compound and block their entry in to the cell. Gentamycin, Hygromycin B, Kanamycin, Neomycin, Streptomycin, G418, Tobramycin. Proteins that allow selection with nucleic acid metabolism inhibitors like Rifampicin, Mitomycin C, Nalidixic acid, Doxorubicin HCl, 5-Flurouracil, 6-Mercaptopurine, Antimetabolites, Miconazole, Trimethoprim, Methotrexate, Metronidazole, Sulfametoxazole are also examples for reporter proteins.

In a preferred embodiment of the present invention the cell or host cell is selected from a mammalian, insect, nematode, plant, yeast, protist cell, non-pathogenic Gram-positive or Gram-negative bacteria, non-pathogenic archaebacteria or non-pathogenic protozoa. In another preferred embodiment of the present invention the cell is a pathogenic organism selected from the group consisting of yeast, gram-positive bacteria, gram-negative bacteria, archaebacteria or protozoa.

In yet another preferred embodiment of the present invention, all or a subset of the proteins encoded by the cell are tagged. Tagged proteins may be expressed as stable construct, i.e. integrated into the genome of the cell or transiently from a vector. Preferably, the tagged protein is expressed from its natural position in the genome of the respective organism.

The present invention also relates to a method of producing and/or selecting a compound capable of modulating a nucleic acid binding protein comprising the steps of: (a) conducting a selection of compounds with the nucleic acid binding target protein under conditions allowing an interaction of the compound and the nucleic acid binding protein; (b) removing unspecifically bound compounds; (c) detecting specific binding of compounds to the nucleic acid binding target protein; (d) expressing in a cell, the nucleic acid binding protein and providing in trans the coding sequence of at least one indicator gene, wherein said coding sequence is under control of the target sequence of the nucleic acid binding protein; (e) adding a candidate compound to the cell of step (d); (f) determining the amount or activity of the indicator protein, wherein a reduced or increased amount of indicator protein is indicative of compounds, capable of modulating the activity of the nucleic acid binding protein; and (g) selecting compounds capable of modulating the activity of the nucleic acid binding protein. The compound identified by this method is an interaction partner of said biomolecule. Preferably, said compounds are (poly)peptides or derivatives thereof.

In a preferred embodiment of the present invention, particularly in cases when the biomolecule is the Tet repressor, said compound is any of the (poly)peptides encoded by the nucleic acid molecules of the present invention or a derivative thereof. Derivatives may be (poly)peptides with substitutions, deletions or additions in order to improve the (poly)peptide's affinity and/or specificity to the biomolecule. In addition, said compounds may contain chemical modifications in order to improve the solubility and/or cellular uptake of the compound. Such modifications include, but are not limited to (i) esterification of carboxyl groups, or (ii) esterification of hydroxyl groups with carbon acids, or (iii) esterification of hydroxyl groups to, e.g. phosphates, pyrophosphates or sulfates or hemi succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl groups to ketales, acetales, or (xiv) N-acetylation to amides, phenylcarbamates, or (xv) synthesis of Mannich bases, imines, or transformation of ketones or aldehydes to Schiffs bases, oximes, acetales, ketales, enolesters, oxazolidines, thiozolidines or combinations thereof.

The present invention also relates to a nucleic acid molecule encoding a (poly)peptide comprising the sequence (a) Met - Trp - Thr - Trp - Asn - Ala - Tyr - Ala - Phe - Ala - Ala - Pro - Ser - Gly - Gly - Gly - Ser (SEQ ID NO: 1); (b) Trp - Thr - Trp - Asn - Ala - Tyr - Ala - Phe - Ala - Ala - Pro - Ser - Gly - Gly - Gly - Ser (SEQ ID NO: 2); (c) Trp - Thr - Trp - Asn - Ala - Tyr - Ala - Phe - Ala - Ala - Pro - Ser (SEQ ID NO: 3); (d) Ser - Gly - Gly - Ala - Trp - Thr - Trp - Asn - Ala - Tyr -Ala - Phe - Ala - Ala - Pro - Ser - Gly - Gly - Gly - Ser (SEQ ID NO: 4); (e) Ser - Gly - Gly - Ala - Trp - Thr - Trp - Asn - Ala - Tyr - Ala - Phe - Ala - Ala - Ala - Ser - Gly - Gly - Gly - Ser (SEQ ID NO: 5); (f) Ser - Gly - Gly - Ala - Trp - Thr - Trp - Asn - Ala - Tyr - Ala - Phe - Ala - Ala - Pro - Ala - Gly - Gly - Gly - Ser (SEQ ID NO: 6); (g) Ser - Gly - Gly - Ala - Trp - Thr - Trp - Asn - Ala - Tyr - Ala - Phe - Ala - Ala - Pro - Ser - Gly - Arg - Gly - Ser (SEQ ID NO: 7); (h) Ser - Gly - Gly - Ala - Trp - Thr - Trp - Asn - Ala - Tyr - Ala - Phe - Ala - Ala - Pro - Ser - Asp - Gly - Gly - Leu (SEQ ID NO: 8); (i) Ser - Gly - Gly - Ala - Trp - Thr - Trp - Asn - Ala - Tyr - Ala - Phe - Ala - Ala - Pro - Ser - Gly - Glu - Gly - Ser (SEQ ID NO: 9); (j) Ser - Gly - Gly - Ala - Trp - Thr - Trp - Asn - Ala - Tyr - Ala - Phe - Ala - Ala - Pro - Ser - Gly - Gly - Gly - Trp (SEQ ID NO: 10); (k) Ser - Gly - Gly - Ala - Trp - Thr - Trp - Asn - Ala - Tyr - Ala - Phe - Ala - Ala - Pro - Ser - Gly - Gly - Cys - Ser (SEQ ID NO: 11); (I) Ser - Gly - Gly - Ala - Trp - Thr - Trp - Asn - Ala - Tyr - Ala - Phe - Ala - Ala - Pro - Ser - Gly - Gly - Asp - Ser (SEQ ID NO: 12); (m) Ser - Gly - Gly - Ala - Trp - Thr - Trp - Asn - Ala - Tyr - Ala - Phe - Ala - Ala - Pro - Ser - Gly - Gly - Arg - Ser (SEQ ID NO: 13); (n) Ser - Gly - Gly - Ala - Trp - Thr - Trp - Asn - Ala - Phe - Ala - Phe - Ala - Ala - Pro - Ser - Gly - Gly - Gly - Ser (SEQ ID NO: 14); or a nucleic acid molecule, the complementary strand of which hybridizes under stringent conditions to the nucleic acid molecule of any one of (a) to (n), wherein said nucleic acid molecule encodes an interaction partner which is capable of modulating the activity of a nucleic acid binding protein.

The term "hybridizes under stringent conditions", as used in the description of the present invention, is well known to the skilled artisan and corresponds to conditions of high stringency. Appropriate stringent hybridization conditions for each nucleic acid sequence may be established by a person skilled in the art on well-known parameters such as temperature, composition of the nucleic acid molecules, salt conditions etc.; see, for example, Sambrook et al., "Molecular Cloning, A Laboratory Manual"; CSH Press, Cold Spring Harbor, 1989 or Higgins and Hames (eds.), "Nucleic acid hybridization, a practical approach", IRL Press, Oxford 1985, see in particular the chapter "Hybridization Strategy" by Britten & Davidson, 3 to 15. Stringent hybridization conditions are, for example, conditions comprising overnight incubation at 42° C in a solution comprising: 50% formamide, 5x SSC (750 mM NaCl, 75 mM trisodium citrate), 50 mM sodium phosphate (pH 7.6), 5x Denhardt's solution, 10% dextran sulfate, and 20 micrograms/ml denatured, sheared salmon sperm DNA, followed by washing the filters in 0.1x SSC at about ,65°. Other stringent hybridization conditions are for example 0.2 x SSC (0.03 M NaCl, 0.003Msodium citrate, pH 7) bei 65°C. In addition, to achieve even higher stringency, washes performed following stringent hybridization can be done at higher salt concentrations (e.g. 5X SSC). Note that variations in the above conditions may be accomplished through the inclusion and/or substitution of alternate blocking reagents used to suppress background in hybridization experiments. Typical blocking reagents include, but are not limited to, Denhardt's reagent, BLOTTO, heparin, denatured salmon sperm DNA, and commercially available proprietary formulations. The inclusion of specific blocking reagents may require modification of the hybridization conditions described above, due to problems with compatibility. Also contemplated are nucleic acid molecules encoding an interaction partner of a biomolecule, wherein the interaction partner is capable of modulating the activity said biomolecule and wherein the nucleic acid molecules hybridize to the nucleic acid molecule encoding the biomolecule at even lower stringency hybridization conditions. Changes in the stringency of hybridization and signal detection are, for example, accomplished through the manipulation of formamide concentration (lower_percentages of formamide result in lowered stringency); salt conditions, or temperature. For example, lower stringency conditions include an overnight incubation at 37 degree C in a solution comprising 6X SSPE (20X SSPE = 3M NaCl; 0.2M NaH₂PO₄; 0.02M EDTA, pH 7.4), 0.5% SDS, 30% formamide, 100 µg/ml salmon sperm blocking DNA; followed by washes at 50 degree C with 1XSSPE, 0.1% SDS. In addition, to achieve even lower stringency, washes performed following stringent hybridization can be done at higher salt concentrations (e.g. 5X SSC). Note that variations in the above conditions may be accomplished through the inclusion and/or substitution of alternate blocking reagents used to suppress background in hybridization experiments. Typical blocking reagents include, but are not limited to, Denhardt's reagent, BLOTTO, heparin, denatured salmon sperm DNA, and commercially available proprietary formulations. The inclusion of specific blocking reagents may require modification of the hybridization conditions described above, due to problems with compatibility.

Preferably, said nucleic acid molecule hybridizing to the nucleic acid molecule encoding the interaction partner of a biomolecule has a sequence identity of at least 60%, 65%, 70%,75%, 80%, 85%, 90%, 95%, 96%, 97%, 98%, 99% or 99.5% when compared to the nucleic acid molecule encoding the interaction partner. The term "which is at least 80% identical" as used in the present invention, relates to sequence identity as determined by the Bestfit® program (Wisconsin Sequence Analysis Package, Version 8 for Unix, Genetics Computer Group, University Research Park, 575 Science Drive, Madison, WI 53711). Bestfit® uses the local homology algorithm of Smith and Waterman to find the best segment of homology between two sequences *(Advances in Applied Mathematics* 2:482-489 (1981)). When using Bestfit® or any other sequence alignment program to determine whether a particular sequence is, for instance, 80% identical to a reference sequence, the parameters are set, of course, such that the,percentage of identity is calculated over the full length of the reference nucleotide or amino acid sequence and that gaps in homology of up to 5% of the total number of nucleotides or amino acids in the reference sequence are allowed. The identity between a first sequence and a second sequence, also referred to as a global sequence alignment, may also be determined by using the FASTDB computer program based on the algorithm of Brutlag and colleagues (*Comp*. *App. Biosci.* 6:237-245 (1990)). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identity are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the length of the subject nucleotide sequence, whichever is shorter.

The present invention also relates to a (poly)peptide encoded by the nucleic acid of the present invention. Preferably, the interaction partner of the present invention contains one copy of the peptide sequence encoded by the nucleic acid molecule of the present invention. However, also encompassed by the present invention are (poly)peptides which contain two, three, four, five or more copies of the peptide sequence encoded by the nucleic acid molecules of the present invention or of any interaction partner as defined in the present invention. Also encompassed by the present invention are (poly)peptides with at least 60%, 65%, 70%,75%, 80%, 85%, 90%, 95%, 96%, 97%, 98%, 99% or 99.5% amino acid sequence identity to the (poly)peptides encoded by the nucleic acid molecules of the present invention, wherein these (poly)peptides are capable of interacting with a biomolecule and of modulating its function.

The present invention also relates to an expression vector, comprising an expression control sequence, a multiple cloning site for inserting a gene of interest and the nucleic acid molecule of the present invention, wherein the gene of interest is inserted in-frame with the ORF encoding the peptide. The term "ORF" means "open reading frame" and relates to a nucleotide sequence encoding and capable of expressing a (poly)peptide.

The present invention also relates to a vector comprising the nucleic acid molecule of the present invention. Preferably said vector is a transfer or expression vector selected from the group consisting of pACT2; pAS2-1; pBTM116; pBTM117; pcDNA3.1; pcDNAI; pECFP; pECFP-C1; pECFP-N1; pECFP-N2; pECFP-N3; pEYFP-C1; pFLAG-CMV-5 a, b, c; pGAD10; pGAD424; pGAD425; pGAD427; pGAD428; pGBT9; pGEX-3X1; pGEX-5X1; pGEX-6P1; pGFP; pQE30; pQE30N; pQE30-NST; pQE31; pQE31N; pQE32; pQE32N; pQE60; pSE111; pSG5; pTK-Hyg; pTL1; pTL10; pTL-HA0; pTL-HA1; pTL-HA2; pTL-HA3; pBTM118c; pGEX-6P3; pACGHLT-C; pACGHLT-A; pACGHLT-B; pUP; pcDNA3.1-V5His; pMalc2x;. Said expression vectors may particularly be BAC (bacterial artificial chromosome) and YAC (yeast artificial chromosome) plasmids, cosmids, viruses and bacteriophages used conventionally in genetic engineering that comprise the aforementioned nucleic acid. Preferably, said vector is a gene transfer or targeting vector. Expression vectors derived from viruses such as retroviruses, vaccinia virus, adenovirus, adeno-associated virus, herpes viruses, or bovine papilloma virus, may be used for delivery of the nucleic acid into targeted cell population. Methods which are well known to those skilled in the art can be used to construct recombinant viral vectors; see, for example, the techniques described in Sambrook et al., Molecular Cloning A Laboratory Manual, Cold Spring Harbor Laboratory (1989) N.Y. and Ausubel et al., Current Protocols in Molecular Biology, Green Publishing Associates and Wiley Interscience, N.Y. (1989).

In yet a further preferred embodiment of the invention the vector contains an additional expression cassette for a reporter protein, selected from the group consisting of ß-galactosidase, luciferase, green fluorescent protein and variants thereof.

Preferably, said vector comprises regulatory elements for expression of said nucleic acid molecule. Accordingly, the nucleic acid of the invention may be operatively linked to expression control sequences allowing expression in eukaryotic cells. Expression of said nucleic acid molecule comprises transcription of the sequence of the nucleic acid molecule into a translatable mRNA. Regulatory elements ensuring expression in eukaryotic cells, preferably mammalian cells, are well known to those skilled in the art. They usually comprise regulatory sequences ensuring initiation of transcription and, optionally, a poly-A signal ensuring termination of transcription and stabilization of the transcript, and/or an intron further enhancing expression of said nucleic acid. Additional regulatory elements may include transcriptional as well as translational enhancers, and/or naturally-associated or heterologous promoter regions. Possible regulatory elements permitting expression in eukaryotic host cells are the AOX1 or GAL1 promoter in yeast or the CMV-, SV40-, RSV-promoter (Rous sarcoma virus), CYC1 promotor, CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the nucleic acid molecule. Furthermore, depending on the expression system used leader sequences capable of directing the (poly)peptide to a cellular compartment or secreting it into the medium may be added to the coding sequence of the aforementioned nucleic acid and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an C- or N-terminal identification peptide imparting desired characteristics, e.g., stabilization or simplified purification of expressed recombinant product. In this context, suitable expression vectors are known in the art such as Okayama-Berg cDNA expression vector pcDVI (Pharmacia), pCDM8, pRc/CMV, pcDNA1, pcDNA3, the Echo™ Cloning System (Invitrogen), pSPORT1 (GIBCO BRL) or pCl (Promega).

The present invention also relates to a host cell containing the nucleic acid molecule of the present invention or the expression vector of the present invention. Preferably, the host cell is a eukaryotic or prokaryotic cell. The cell can be the cell of a uni-cellular or multi-cellular organism, which may be pathogenic or non-pathogenic. Preferably, the host cell is selected from mammalian, insect, nematode, plant, yeast, protist cell, gram-positive or gram-negative bacteria, archaebacteria or protozoa.

In a preferred embodiment, the nucleic acid molecule of the present invention is fused in frame to at least one chromosomal sequence encoding a (poly)peptide. In another preferred embodiment, the host cell of the present invention also contains (a) the coding sequence of a reporter protein which is under control of a nucleic acid binding protein; and (b) the coding sequence of the nucleic acid binding protein of (a), wherein said coding sequences are operatively linked to expression control sequences. As a consequence of the fusion of the nucleic acid molecule of the present invention to the chromosomal sequence encoding a (poly)peptide, said (poly)peptide is capable of binding to the nucleic acid binding protein which will result in a modulation of gene expression of the reporter protein controlled by said nucleic acid binding protein. In cases when the nucleic acid binding protein is a repressor, the negative or repressing effect will be suspended so that gene expression can start or proceed. In cases when the nucleic acid binding protein is an enhancer or activator, binding of the fusion protein to the nucleic acid binding protein will result in activation of gene expression and in production of the reporter gene product or protein.

In a preferred embodiment of the present invention's host cell, the nucleic acid binding protein is a repressor of transcription. In another preferred embodiment of the present invention's host cell, the nucleic acid binding protein is an enhancer of transcription. Preferably said enhancer of transcription is cAMP responsive protein (CRP) or AraC.

In a particularly preferred embodiment of the present invention, said nucleic acid binding protein is a (poly)peptide comprising the (poly)peptide sequence of (a) Tet repressor; (b) lac repressor; (c) Xylose repressor, (d) AraC protein; (e) TetR-based T-Rex system; (f) erythromycin-specific repressor MphR(A); (g) Pip (pristinamycin interacting protein); (h) ScbR from *Streptomyces coelicolor*; (i) TraR of *Agrobacterium tumefaciens*; fused to the eukaryotic activation domain p65 of NFκB; or (j) chimeric proteins. Preferably said chimeric proteins consist of (i) Gal4 DNA-binding domain and either (1) a full-length PhyA protein (PhyA-GBD) or (2) the N-terminal photosensory domain of PhyB [PhyB(NT)-GBD] of *Arabidopsis thaliana*; (ii) steroid hormone regulated systems like the GeneSwitch regulatory system from Valentis (www.geneswitch.com), sold by Invitrogen (catalog number K1060-02) and consisting of (1) a Gal4 DNA-binding domain fused to a human progesterone receptor ligand binding domain and an NFκB-derived p65 eukaryotic transcription activation domain and (2) the inducer mifepristone; (iii) Dimerizer systems from ARIAD consisting of (1) a ZFHD1 DNA-binding domain fused to FKBP 12, (2) a modified form of FRAP, termed FRB, fused to the NFκB-derived p65 activation domain and (3) rapamycin, AP22565 or AP12967 as heterodimer-forming agents, as they bind to both (1) and (2).or (iv) Ecdysone-Inducible Expression System (with the Inducing Agents Ponasterone A and Muristerone A) from Invitrogen (catalog numbers K1001-01 K1003-01, K1004-01) containing (1) a modified form of the *Drosophila* ecdysone receptor (VgEcR) fused to a VP16 activation domain, (2) the mammalian homologue RXR of *ultraspiracle,* the natural binding partner of the ecdysone receptor in *Drosophila* and (3) the inducers ponasterone A and muristerone A.

The present invention also relates to an ensemble of host cells of the present invention, wherein said ensemble comprises two or more cells, each of which contain at least one nucleic acid molecule fused in frame to an open reading frame encoding a (poly)peptide. The term "ensemble of host cells" refers to a population of cells, wherein said population contains cells with differently tagged host proteins. Preferably, an individual cell only contains one open reading frame tagged with nucleic acid sequences encoding the interaction partner of the regulatory biomolecule. However, in some cases it may be desirable that 2, 3, 4, 5, 6, 7, 8, 9, or more ORFs contain nucleic acid sequences encoding the interaction partner of the regulatory biomolecule. Preferably, the ensemble or population of cells in their sum are characterized by representing the entire proteome of the host.

In a preferred embodiment of the present invention's host cell, the nucleic acid binding protein is an enhancer or activator of transcription.

In another preferred embodiment, the ensemble of host cells of the present invention contains subpopulations with different open reading frames being fused to said nucleic acid molecule of the present invention. In a more preferred embodiment of the present invention the sum of said open reading frames forms the proteome of the host cell.

The present invention also relates to a non-human animal containing the host cell of the present invention or the ensemble of host cells of the present invention. The term "non-human" relates to vertebrate and invertebrate animals. Preferred animals are *Drosophila,* mouse, rat, rabbit, monkey and cat. Hosts for pathogens can be inoculated intravenously with pathogens (Ji et al., 1999; Nakayama et al., 1998), by intraperitoneal injection (van Deursen et al., 2001), subcutaneous injection (Dubey et al., 2001), injection into the abdomen (Dionne et al., 2003), aerosol infection by inhalation (Schwebach et al., 2002), by infection with natural hosts (van Deursen et al., 2001), or by simple feeding (Dubey et al., 2001).

The present invention also relates to a kit comprising (a) the nucleic acid molecule, (b) the (poly)peptide, (c) the vector and/or (d) the host cell or ensemble of host cells of the present invention and (e) instructions for use; in one or more containers.

Finally, the present invention relates to the use of the (poly)peptide of the present invention, the nucleic acid molecule of the present invention, the expression vector of the present invention or the host cell or ensemble of host cells of the present invention for monitoring expression of a gene.

### Figures:

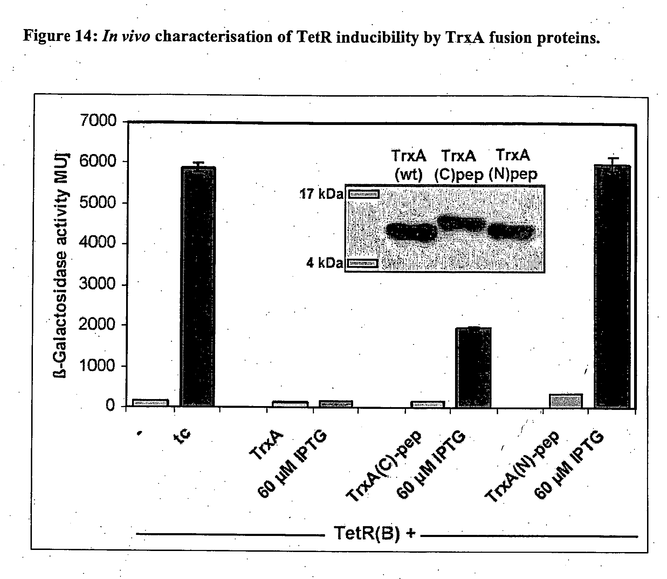

**Figure 1: Experimental procedure for the** ***in vitro*** **selection.** For the phage display (Kay et al., 2001) experiments, an M13 phage bank (Mourez et al., 2001) of about 10⁹ different dodecapeptides was used. After the first round of selection, binding phages were eluted unspecifically by low pH. In round 2, the eluate from round 1 was split into 3 different modes of elution, which were also used in round 3. The output phage titer was determined after each round (shown as pfu/ml for TetR-specific elution). The ≈ 400x increase indicates the enrichment of binding phages. After the third round individual clones were isolated, sequenced and characterised for TetR-binding by ELISA.
**Figure 2: Example for** ***in vitro*** **selected sequences.** The first five candidates were obtained by unspecific elution using a glycine-containing buffer at pH2.2. The second five, containing the peptide pepBs1, were obtained by specific elution with 4µM TetR(B).

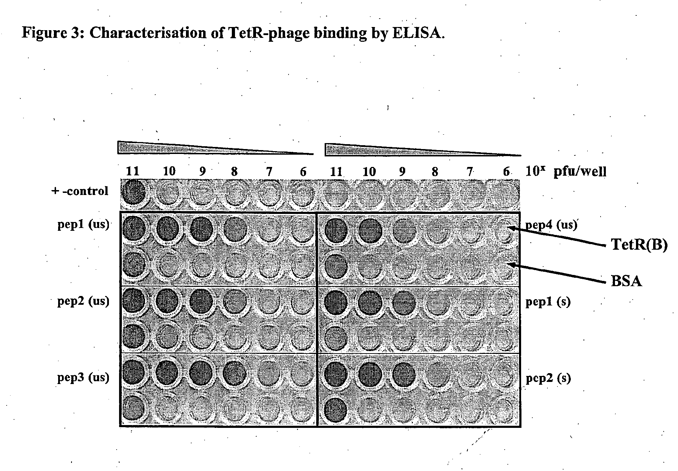

**Figure 3: Characterisation of TetR-phage binding by ELISA.** To test for TetR-phage-interactions (Kay et al., 2001), 96-well microtiter plates were coated with TetR(B) or bovine serum albumin (BSA) as negative control. M13-phage clones were then added in increasing concentrations. After incubation and several washing steps, a monoclonal Anti-M13 antibody coupled to horseradish peroxidase was added. TetR-M13 interaction was detected by dye formation, after adding the substrate ABTS (2',2'-Azino-bis(ethylbenzthiazolin-6-sulfonic acid). The abbreviations (s) and (us) represent specific and unspecific elution, respectively, pfu represents "plaque forming units".

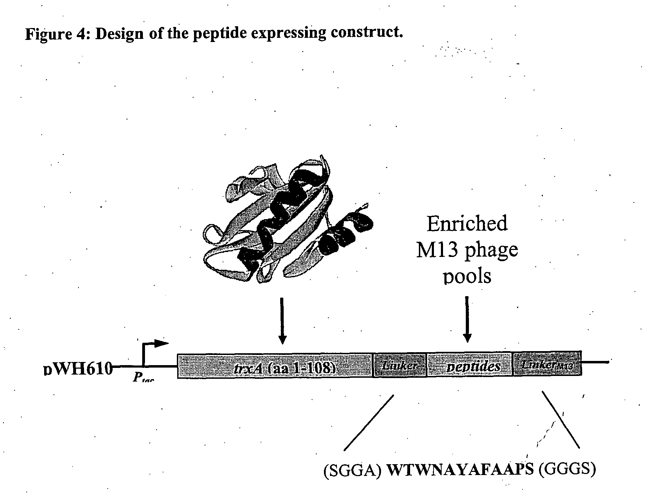

**Figure 4: Design of the peptide expressing construct.** The *E*. *coli* protein thioredoxin A (TrxA) is a small and highly soluble cytoplasmic protein which serves as the carrier (Park and Raines, 2000) for the *in vitro* selected peptides. The *trxA* gene was cloned under the expression control of *P*_{*tac*} (Ettner et al., 1996). The *in vitro* selected peptides were fused with a spacer to the solvent exposed C-terminus of TrxA. They also contained the M13 linker present in the initial selection. The expression of the TrxA-peptide fusion proteins is induced by addition of IPTG.

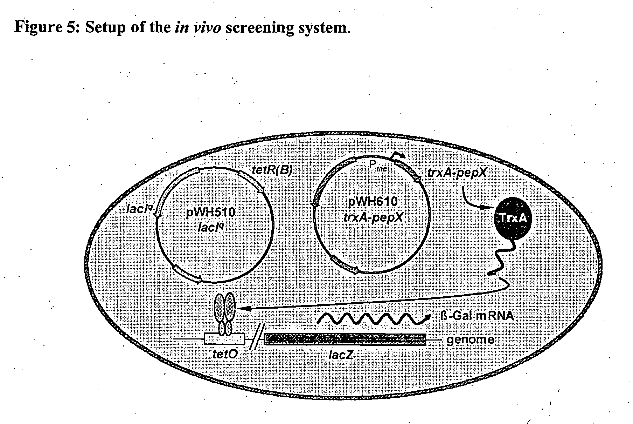

**Figure 5: Setup of the** ***in vivo*** **screening system.** The screening was carried out in an *E*. *coli* strain containing the *lacZ* gene under Tet control (Smith and Bertrand, 1988). pWH510*lacl*^{q} expresses TetR constitutively (Altschmied et al., 1988). TetR binds to its operator sequence and thereby prevents transcription of the reporter gene. pWH610*trxA*-*pepX* encodes a fusion protein (*pepX* stands for any *in vitro* selected peptide). Its expression is induced by addition of IPTG (Ettner et al., 1996). If a peptide fused to thioredoxin binds and induces TetR, ß-Galactosidase is expressed. This can be detected on McConkey plates and quantified in LacZ assays.

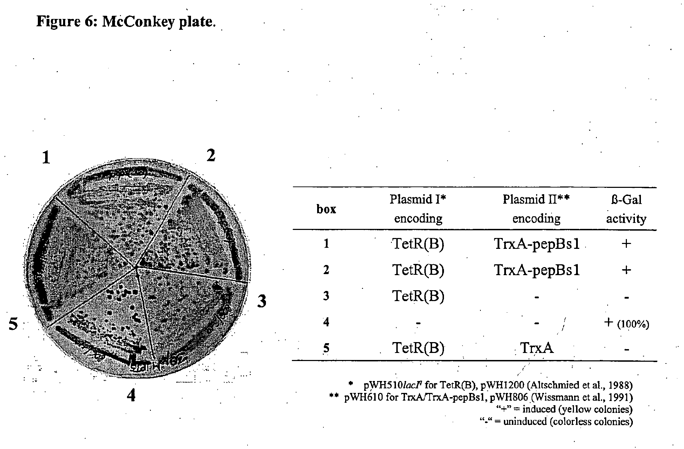

**Figure 6: McConkey plate.** The *E*. *coli* strain DH5α-λ*tet*50 was transformed with plasmids shown in the table above and streaked out to single colonies on McConkey plates (+ 30 µM IPTG).

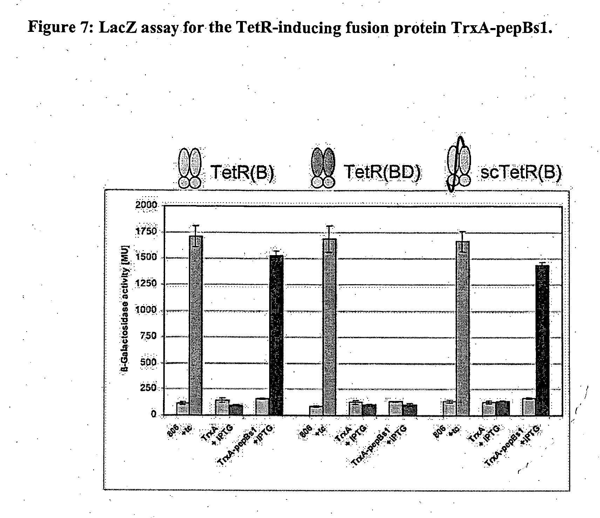

**Figure 7: LacZ assay for the TetR-inducing fusion protein TrxA-pepBs1.** This assay was carried out using the *in vivo* system from figure 6. The repressed state is symbolized with light grey bars, the tc-induced state with dark grey bars. TrxA without the selected peptide was used as a negative control. Upon induction with 125 µM IPTG, the *in vitro* selected peptide pepBs1 (as fusion to TrxA) induces TetR(B), which was used as target during the selection. In contrast, a TetR(BD) chimera, in which the protein core containing the inducer-binding and dimerisation domains of TetR(B) (shown in light grey in the cartoon) is replaced by the sequence of TetR from class D (shown in dark grey in the cartoon) is not induced. The single chain TetR (scTetR) (Krueger et al., 2003) of class B, in which the C-terminus of one monomer is linked to the N-terminus of the other monomer, is also induced.

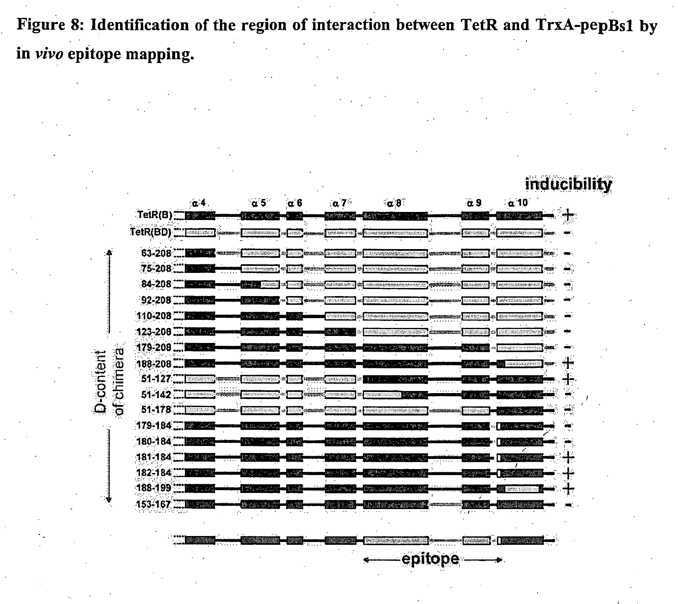

**Figure 8: Identification of the region of interaction between TetR and TrxA-pepBs1 by in** ***vivo*** **epitope mapping.** To delimit the region of interaction, in *vivo* epitope mapping was carried out. For that purpose, we constructed a set of 17 TetR(BD) chimeras (Schnappinger et al., 1998), in which different parts of α4 to α10 were replaced by the TetR(D) sequence, and characterised them for inducibility *in vivo.* We exploited the fact that TetR(B) is inducible by the peptide, but TetR(BD) is not - a B-D exchange which leads to a loss of induction thus indicates the importance of at least part of the replaced region. The *in vivo* inducibility of a chimera is marked with + or -. Characterisation of these chimeras resulted in a defined region of interaction spanning from the beginning of α8 up to the first amino acids of α10.

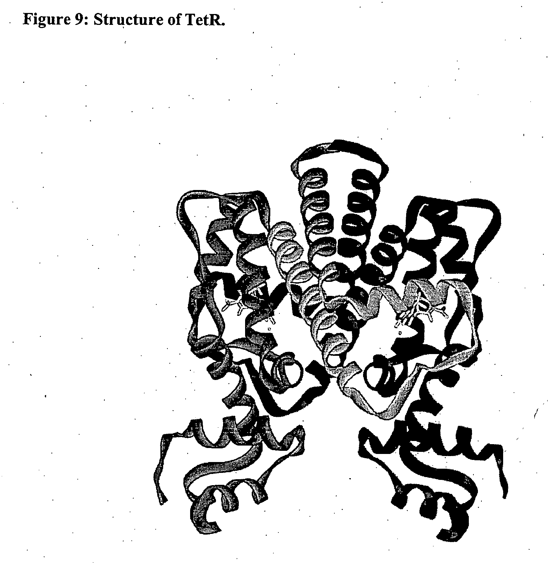

**Figure 9: Structure of TetR.** One monomer is shown in black, the other in dark grey. Mg²⁺-Tetracyclines are shown as white stick models. A class B to D exchange in the portion depicted in light grey leads to a loss of inducibility and represents the region involved in induction by the peptide pepBs1.

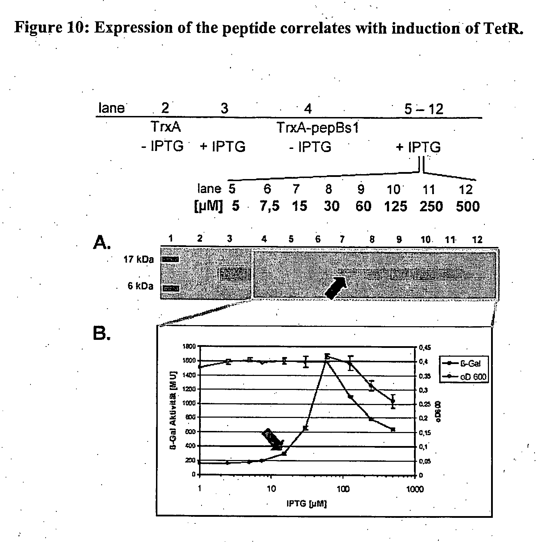

**Figure 10: Expression of the peptide correlates with induction of TetR.** For this experiment, cells were grown under uninduced and induced conditions using IPTG concentrations between 1 µM and 500 µM. The viability was determined by oD₆₀₀ measurement and crude lysates were prepared to detect the expression level of the fusion protein. LacZ measurements were carried out to determine the induction of TetR.
   **A. Expression of the peptide fusion**. Lane 2 of the Western blot shows TrxA uninduced, lane 3 expressed TrxA after induction with 500 µM IPTG. Lane 4 to 12 show the expression of the fusion protein TrxA-pepBs1 using increasing IPTG concentrations (see table). **B. Induction of TetR** (LacZ data compared to oD₆₀₀ and [IPTG]). The ß-Galactosidase activity is depicted as a black curve. TetR induction is first observed using 15 µM IPTG to induce (marked with a black arrow) the expression of TrxA-pepBs1. Maximum induction was reached at an IPTG concentration of 60 µM. Up to that point, the expression level of TrxA-pepBs1 correlates with the induction of TetR. Higher concentrations of the fusion protein appear to have a negative effect on cell viability, as can be seen by the decrease of the oD₆₀₀ (see grey curve). Concomitantly, the steady state level of the fusion protein is diminished and the induction of TetR is also reduced.

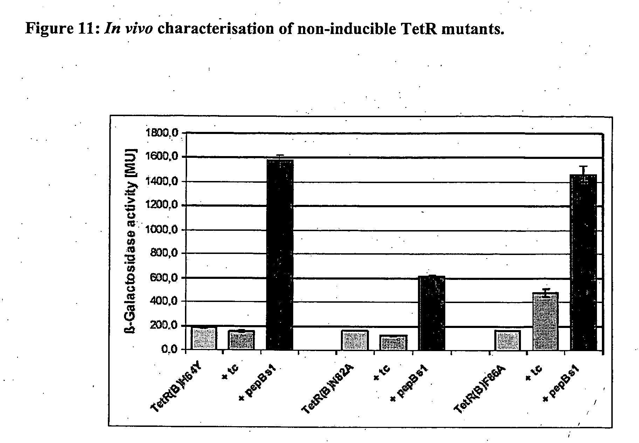

**Figure 11:** ***In*** **vivo characterisation of non-inducible TetR mutants.** The observation that all TetR(BD) chimeras shown in figure 9 are inducible by tc, but only a few are inducible with the peptide, provided the first indication that induction of TetR by the peptide is mechanistically different from induction by tc. To substantiate this assumption we analyzed TetR mutants (Müller et al., 1995) which carry a single amino acid exchange in tc-contacting residues *in vivo.* The repression of the reporter gene by TetR is shown with light grey bars, the induction with tc with dark grey bars. The mutants H64Y, N82A and F86A were not or only slightly inducible with tetracycline but either fully or at least partially inducible with the peptide (see black bars).
**Figure 12: Position of the amino acids H64, N82 and F86 relative to tetracycline and the interaction epitope.** The region of TetR-peptide interaction mapped for each monomer is shown in light and dark grey, respectively. Tc is depicted as light grey stick model. The residues in the mutants which showed the phenotype of being inducible by the peptide but not by tc, when exchanged, are grouped around the A-ring of tc.
**Figure 13:** Amino acids contacting tc either directly or indirectly via the hydrated magnesium cation (tc is shown as light grey stick model).
**Figure 14:** ***In vivo*** **characterisation of TetR inducibility by TrxA fusion proteins.**
   **A.***ß-Galactosidase measurement.* This assay is carried out in *E. coli* DH5*α-λtet*50 which carries the *lacZ* gene under Tet-control as a single copy integrated into the *E. coli* genome. The first bar shows the repressed state when TetR binds to its operator sequence *tet*O. After addition of tetracycline, TetR is induced and β-Galactosidase is expressed. TrxA fusion proteins are under transcription control of *P*_{tac} and induced by addition of IPTG. TrxA without the fused peptide was used as a control and is not capable of inducing TetR. After addition of 60 µM IPTG, the C-terminal TrxA-peptide fusion induces TetR about 14-fold. The N-terminal fusion reaches a factor of induction of about 40 and is, thus, more effective.
   **B.** *Western Blot analysis.* A monoclonal TrxA antibody was used to detect TrxA and TrxA fusion proteins in samples which were used simultaneously for carrying out β-Galactosidase activity measurements and for preparing crude lysates. The data indicates that all proteins were expressed at the same level and the increase of induction seen for the N-terminal fusion is not due to a higher protein amount.

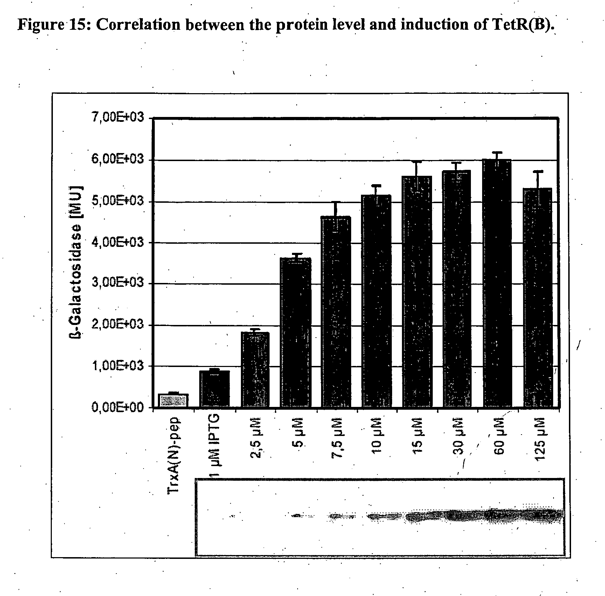

**Figure 15: Correlation between the protein level of PepBs1-TrxA and induction of TetR(B).**
   β-Galactosidase measurement and Western blot analysis were carried out as explained in figure 14. The N-terminal TrxA-peptide fusion was induced using IPTG concentrations ranging from 1 µM up to 125 µM. The Western blot data shows that the amount of fusion protein correlates to the induction of TetR(B) as reflected in the increase in β-Galactosidase activity. A maximum of induction was reached using 15 µM IPTG.
**Figure 16: Comparison of constitutive expression systems leading to tow and medium steady-state levels of TetR and their influence on the induction profile by PepBs1-TrxA.**
   β-Galactosidase measurements were carried out as detailed in figures 14 and 15 with the exception of TetR(B) being expressed from different plasmids. pWH510*lacl*^{q} expresses TetR constitutively at a low level (shown in dark grey bars) whereas pWH1411*lacl*^{q} expresses TetR constitutively at a medium level (shown in light grey bars). In the low expressing system, induction of TetR is observed even for very low IPTG concentrations which is equivalent to a low protein level of the TrxA-peptide fusion (see figure 15). The maximum β-Galactosidase activity is reached at IPTG concentrations of 15 µM or higher. The higher amount of TetR in the medium expression system leads to a delayed increase in β-Galactosidase activity requiring the presence of higher amounts of the fusion protein for full induction. Consequently, the maximum of β-Galactosidase activity is reached at 60 µM IPTG and higher.
**Figure 17: Comparison of TetR(B) induction by C- and N-terminal TrxA-peptide fusions.**
   β-Galactosidase measurements were carried out as explained in figure 14 and using the low-level TetR-expression system pWH510*lacl*^{q}. While induction of TetR by the C-terminal fusion is characterised by a curve having a weakly ascending slope, induction of TetR by the N-terminal fusion is characterised by a curve profile with a steeply ascending slope. In addition, the N-terminal fusion leads to an about 3-fold higher induction compared to the C-terminal fusion.

The examples illustrate the invention

### Example 1: In vitro selection of TetR-binding peptides

Several different experimental approaches can be employed to identify novel ligands for proteins. The screening of low molecular weight compound libraries permits the identification of small molecules that bind to a given target protein. These libraries can consist of a diverse collection of natural products or, alternatively, contain a large set of synthetic compounds generated by combinatorial chemistry. A completely different approach is the use of *in vitro* evolution methods (Lin and Cornish, 2002). These allow the selection of biological macromolecules that display affinity to a defined target molecule. The SELEX method (**S**ystematic **E**volution of **L**igands by **EX**ponential Enrichment) allows the isolation of nucleic acids, RNA or DNA, that bind a defined target with high affinity and specificity (Tuerk and Gold, 1990). Several methods have been developed to select peptide ligands. Two more recent developments are "mRNA-display" or "ribosome display" (Wilson et al., 2001). A more common method is "phage display" (Giannattasio and Weisblum, 2000) which was also used in our selection for TetR-binding peptides.

Phage display: The basis for most phage display applications is the filamentous phage M13 from *Escherichia coli.* A surface protein, gplll which is present in five copies per phage and important for infection of the bacteria can tolerate N-terminal insertions to a certain degree. Since the N-terminus of this protein is solvent-exposed, the insertions are presented on the surface of the phage (Kay et al., 2001). The commercially available phage bank Ph.D.-12™ from New England Biolabs was used to select for TetR binding peptides. It contains ~10⁹ different dodecapeptides fused via a flexible linker of four amino acids to the N-terminus of the gpIII protein. The *in vitro* selection was performed by coating polystyrene tubes (NUNC Maxisorb) with purified Tet repressor protein from class B, TetR(B) (Ettner et al., 1996). The tubes were then incubated with the pool of M13 phages, washed several times and TetR(B)-bound phages were eluted either specifically by addition of TetR(B) or unspecifically by lowering the pH value. Three selection cycles were performed and the enrichment of TetR-binding phages was monitored by determining the M13 titer after each round (Figure 1). Individual M13 clones were picked and sequenced after the third selection round (Figure 2).

Immunological detection of the M13-TetR-interaction: Binding of individual M13 clones to TetR was determined by ELISA (Enzyme Linked Immunosorbent Assay). The phages were amplified, precipitated and resuspended in a small volume. 96-well microtiter-plates were coated with TetR(B), then blocked with Bovine Serum Albumin, followed by incubation with increasing amounts of M13 phages from different isolates, several washes with buffer and, finally, incubated with an M13-specific monoclonal antibody covalently coupled to horseradish peroxidase. Addition of ABTS (2',2'-Azino-bis(ethylbenzthiazolin-6-sulfonic acid) as substrate permits the spectophotometric detection of phage-binding to TetR. The degree of absorption serves as a quantitative indicator of phages bound to the target protein (Kay et al., 2001). For the peptide pepBs1. which will be discussed in more detail in the following sections, the A_{TetR}/A_{BSA} factor determined with the phage-dilution containing 10¹⁰ pfu was 34 (Figure 3).

### Example 2: In vivo screening for TetR-inducing peptides

Establishing an *in vivo* screen in *Escherichia coli*: After having obtained and identified several TetR-binding peptides, the next goal was to isolate peptides that could induce TetR(B). Since small oligopeptides are rapidly degraded intracellulary by proteases, the peptide-encoding sequences were cloned as C-terminal fusions to the *Escherichia coli* protein thioredoxin, an established carrier protein for peptides (Park and Raines, 2000). The thioredoxin fusion proteins were expressed by a *tac* promoter under control of Lac repressor and, thus, inducible by addition of IPTG (Isopropyl-β-thiogalactoside) (Ettner et al., 1996) (see Figure 4 for illustration). TetR(B) was expressed constitutively at a low level (Altschmied et al., 1988). The indicator strain was *E. coli* DH5α(λ*tet*50) containing the phage λ*tet*50 (Smith and Bertrand, 1988) integrated in single copy into the *E. coli* genome. This phage contains a *tet*A-*lac*Z transcriptional fusion. Expression of β-galactosidase is, thus, regulated by TetR that binds to *tetO* sequences located within the promoter (Figure 5). The pool of TetR-binding peptides is screened by plating transformed colonies on MacConkey agar containing IPTG. An inducing Trx-peptide fusion protein will lead to the expression of β-galactosidase, resulting in an acidification of the medium surrounding the colony which can be detected by its yellow color (Figure 6, sectors 1, 2 and 4).

Cloning of individual candidates: Individual candidates identified by sequencing were cloned as C-terminal fusions to TrxA, transformed into the reporter strain DH5α(λ*tet*50) and the respective β-galactosidase activities determined. The following controls were also included in the measurements: To define the regulatory window, both the repressed state (0% - TetR binds to *tet*O) and the fully induced state in the presence of tc (100% - TetR dissociates from *tet*O) were determined. To exclude that thioredoxin is involved in the TetR-peptide interaction, a plasmid expressing thioredoxin without a peptide fusion was also assayed in the presence and absence of IPTG. The β-galactosidase activities of the individual candidates cloned were also determined in the presence and absence of IPTG.

β-Galactosidase activity assays: A TetR-inducing Thioredoxin-peptide fusion protein was identified in the pool of cloned TetR-binding peptides. The fusion protein is specific for TetR(B), since a chimeric repressor, TetR(B/D), is not induced in the presence of Trx-pepBs1. This chimera consists of the DNA-binding domain (residues 1-50) from TetR(B) and the protein core with the inducer-binding and dimerization domains from TetR(D). The sequence identity between the two classes is 63 % at the amino acid level. A single-chain TetR(B) variant in which both subunits of TetR are fused to a monomer by a flexible protein linker connecting the C-terminus of one subunit to the N-terminus of the other was also induced by TrxA-pepBs1 (Figure 7). This suggests than an interaction of the peptide with the dimerization surface of TetR and subsequent induction of TetR by dissociation appear unlikely. The sequence of the peptide pepBs1, with linker elements is shown in Figure 4.

### Example 3: Identification of the interaction site between TetR and the inducing peptide

We isolated the interaction site of TrxA-pepBs1 with TetR(B) by *in vivo* epitope mapping, taking advantage of the observation that TetR(B/D) is not induced by the peptide. We therefore constructed chimeric repressors in which *tetR*(B) sequences are exchanged to different extents by the corresponding sequences from *tetR*(D) (Schnappinger et al., 1998) and determined their *in vivo* inducibility by TrxA-pepBs1. Figures 8 and 9 summarize the results obtained. The inducibility profile shows that interactions between repressor and peptide are confined to the region from helix α8 to residue 182 in helix α10. The loop connecting the helices α9 and α10 also. appears to be important, as chimeras containing *tetR*(D) sequences at residues 179-184 and 180-184 are not inducible by TrxA-pepBs1.

### Example 4: Analysis of TetR mutants with an induction-deficient phenotype

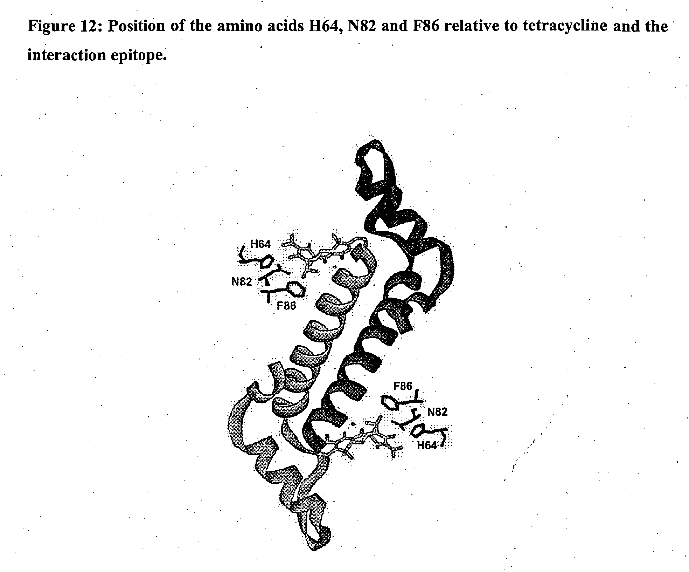

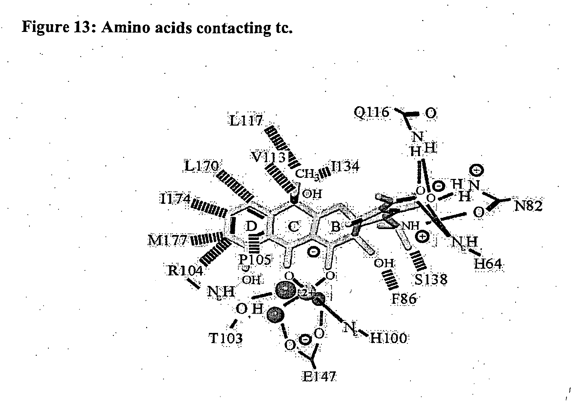

To demonstrate that induction mediated by pepBs1 follows a novel mechanism, different than that of tetracycline, we cloned induction mutants of TetR(B) (Müller et al., 1995) into the *in vivo* test system. The mutants contain single residue exchanges that lead to an induction-deficient phenotype. The mutant TetR(B)HY64 with an exchange of the histidine residue at position 64 by tyrosine is not inducible by tetracycline. β-Galactosidase activity assays show, however, that it is still inducible by TrxA-pepBs1 (see figures 11 to 13 and table 1).

### Example 5: The inducing tag is also active as N-terminal fusion.

The versatility of the inducing tag as a marker for protein presence would be greatly enhanced if it were not confined to C-terminal protein fusions , as these need not be active with all proteins (Huh et al., 2003). We therefore fused the inducing peptide with the M13 linker element genetically to the N-terminus of thioredoxin A from *E*. *coli.* We expressed this fusion protein from pWH610 in the same *in vivo* assay system as shown in Figure 5. Fusion protein expression was induced by addition of IPTG to a final concentration of 60 µM, β-galactosidase activities were measured and the fusion protein amounts were determined by Western blotting of crude extracts. The results shown in Figures 14 and 17 clearly demonstrate that the N-terminal fusion is not only active, but more active than the C-terminal fusion. Its 40-fold induction are higher than the 14-fold induction obtained with the C-terminal fusion. This is not due to higher steady-state amounts of the fusion protein, as is evident from the Western blot data, but rather reflects an intrinsic activity of the protein

### Example 6: The expression level of the tagged protein correlates with reporter gene activity.

Quantification of the tagged protein requires a correlation between the measured reporter gene activity and the amount of the tagged protein expressed. To test if this is the case, we induced expression of the TrxA-peptide fusion protein to different extents by varying the amounts of the inducer IPTG. We then measured the respective β-galactosidase activity and determined the corresponding amounts of the TrxA-peptide fusion protein by Western blotting with a monodonal antibody against TrxA. Figures 10 and 15 show that higher amounts of the TrxA-peptide fusion protein lead to higher amounts of β-galactosidase activity. In Figure 15, a plateau is reached at an IPTG concentration of 15 µM, and β-galactosidase activity is fully induced. This is most likely due to all TetR present in the cell having been bound by the tagged protein. The resolution window can be shifted by increasing the intracellular steady-state amount of TetR. For example, pWH1411 expresses higher amounts of TetR than pWH510 (Wissmann et al., 1991). Consequently, in the presence of pWH1411, higher amounts of the fusion protein are needed to reach the same β-galactosidase activities, as in the presence of pWH510 (compare 2.5 µM IPTG for pWH510 with 15 µM IPTG for pWH1411), and the plateau corresponding to full induction of β-galactosidase activity is only reached at an IPTG concentration of 60 µM (Figure 16).

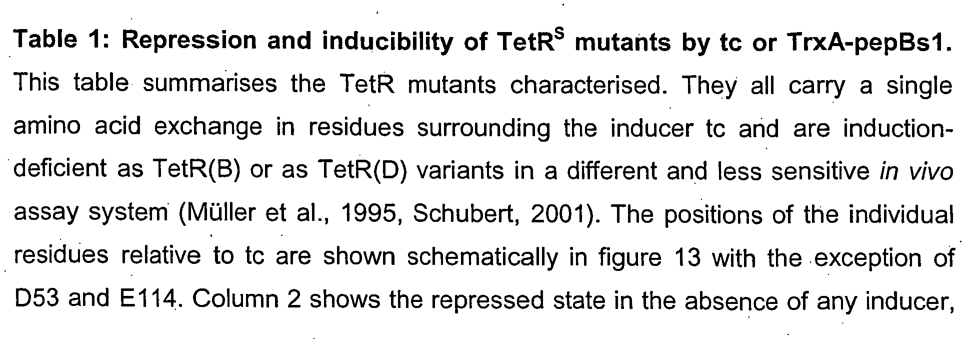

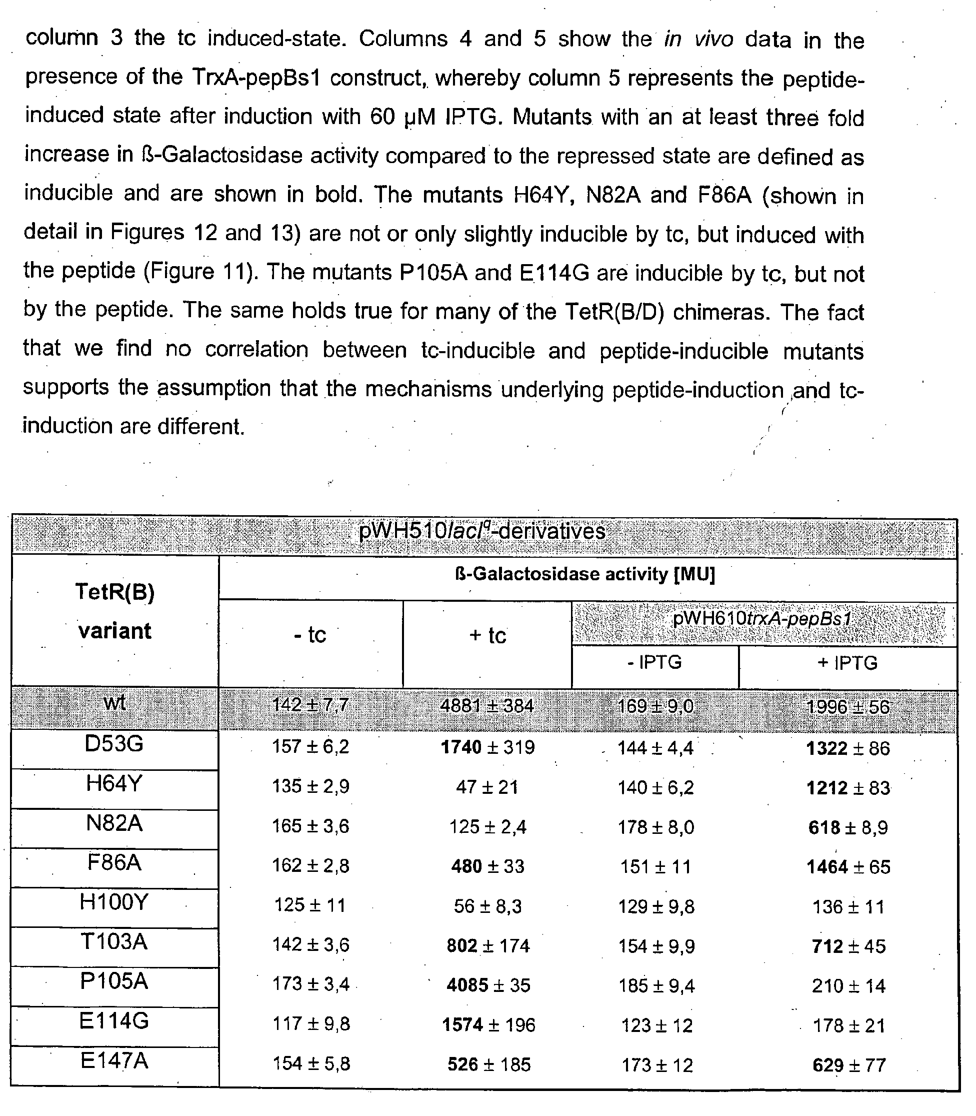

### References

Altschmied, L., Baumeister, R., Pfleiderer, K. and Hillen, W. (1988). A threonine to alanine exchange at position 40 of Tet repressor alters the recognition of the sixth base pair of *tet* operator from GC to AT. EMBO J *7*, 4011-4017.
Aung-Hilbrich, L. M., Seidel, G., Wagner, A., and Hillen, W. (2002). Quantification of the influence of HPrSer46P on CcpA-*cre* interaction. J Mol Biol *319*, 77-85.
Baron, U., Gossen, M. and Bujard, H. (1997). Tetracycline-controlled transcription in eukaryotes: novel transactivators with graded transactivation potential. Nucleic Acids Res *25*, 2723-2729.
Breaker, R. R. (2002). Engineered allosteric ribozymes as biosensor components. Curr Opin Biotechnol *13*, 31-39.
Calnan, B. J., Biancalana, S., Hudson, D., and Frankel, A. D. (1991). Analysis of arginine-rich peptides from the HIV Tat protein reveals unusual features of RNA-protein recognition. Genes Dev *5*, 201-210.
Chan, K., Knaak, T., Satkamp, L., Humbert, O., Falkow, S., and Ramakrishnan, L. (2002). Complex pattern of *Mycobacterium marinum* gene expression during long-term granulomatous infection. Proc Natl Acad Sci USA *99*, 3920-3925.
Cherepanov, P. P., and Wackernagel, W. (1995). Gene disruption in *Escherichia coli*: TcR and KmR cassettes with the option of Flp-catalyzed excision of the antibiotic-resistance determinant. Gene *158*, 9-14.
Datsenko, K. A., and Wanner, B. L. (2000).,One-step inactivation of chromosomal genes in *Escherichia coli* K-12 using PCR products. Proc Natl Acad Sci USA *97*, 6640-6645.
Deiwick, J., Rappl, C., Stender, S., Jungblut, P. R., and Hensel, M. (2002). Proteomic approaches to *Salmonella* Pathogenicity Island 2 encoded proteins and the SsrAB regulon. Proteomics *2*, 792-799.
Dionne, M. S., Ghori, N., and Schneider, D. S. (2003). *Drosophila melanogaster* is a genetically tractable model host for *Mycobacterium marinum.* Infect Immun 71, 3540-3550.
Dubey, J. P., Lindsay, D. S., Kwok, O. C., and Shen, S. K. (2001). The gamma interferon knockout mouse model for sarcocystis neurona: comparison of infectivity of sporocysts and merozoites and routes of inoculation. J Parasitol 87, 1171-1173.
Eichenbaum, Z., Federle, M. J., Marra, D., de Vos, W. M., Kuipers, O. P., Kleerebezem, M., and Scott, J. R. (1998). Use of the lactococcal *nisA* promoter to regulate gene expression in gram-positive bacteria: comparison of induction level and promoter strength. Appl Environ Microbiol *64*, 2763-2769.
El Fakhry, Y., Ouellette, M., and Papadopoulou, B. (2002). A proteomic approach to identify developmentally regulated proteins in *Leishmania infantum.* Proteomics 2, 1007-1017.
Epshtein, V., Mironov, A. S., and Nudler, E. (2003). The riboswitch-mediated control of sulfur metabolism in bacteria. Proc Natl Acad Sci USA *100*, 5052-5056.
Eriksson, S., Lucchini, S., Thompson, A., Rhen, M., and Hinton, J. C. (2003). Unravelling the biology of macrophage infection by gene expression profiling of intracellular *Salmonella enterica.* Mol Microbiol *47*, 103-118.
Ettner, N., Müller, G., Berens, C., Backes, H., Schnappinger, D., Schreppel, T., Pfleiderer, K., and Hillen, W. (1996). Fast large-scale purification of tetracycline repressor variants from overproducing *Escherichia coli* strains. J Chromatogr A *742*, 95-105.
Fussenegger, M., Morris, R. P., Fux, C., Rimann, M., von Stockar, B., Thompson, C. J., and Bailey, J. E. (2000). Streptogramin-based gene regulation systems for mammalian cells. Nat Biotechnol *18*, 1203-1208.
Geissendörfer, M., and Hillen, W. (1990). Regulated expression of heterologous genes in *Bacillus subtilis* using the Tn*10* encoded *tet* regulatory elements. Appl Microbiol Biotechnol 33, 657-663.
Ghaemmaghami, S., Huh, W. K., Bower, K., Howson, R. W., Belle, A., Dephoure, N., O'Shea, E. K. and Weissman, J. S. (2003). Global analysis of protein expression in yeast. Nature *425*, 737-741.
Glannattasio, R. B. and Weisblum, B. (2000). Modulation of *erm* methyltransferase activity by peptides derived from phage display. Antimicrob Agents Chemother *44*, 1961-1963.
Gossen, M. and Bujard, H. (1992). Tight control of gene expression in mammalian cells by tetracycline-responsive promoters. Proc Natl Acad Sci USA *89*, 5547-5551.
Gosser, Y., Hermann, T., Majumdar, A., Hu, W., Frederick, R., Jiang, F., Xu, W., and Patel, D. J. (2001). Peptide-triggered conformational switch in HIV-1 RRE RNA complexes. Nat Struct Biol *8*, 146-150.
Guina, T., Purvine, S. O., Yi, E. C., Eng, J., Goodlett, D. R., Aebersold, R., and Miller, S. I. (2003). Quantitative proteomic analysis indicates increased synthesis of a quinolone by *Pseudomonas aeruginosa* isolates from cystic fibrosis airways. Proc Natl Acad Sci USA *100,* 2771-2776.
Gygi, S. P., Corthals, G. L., Zhang, Y., Rochon, Y., and Aebersold, R. (2000). Evaluation of two-dimensional gel electrophoresis-based proteome analysis technology. Proc Natl Acad Sci USA *97*, 9390-9395.
Hanash, S. (2003). Disease proteomics. Nature *422*, 226-232.
Harada, K., Martin, S. S., and Frankel, A. D. (1996). Selection of RNA-binding peptides *in vivo*. Nature *380*, 175-179.
Harada, K., Martin, S. S., and Frankel, A. D. (1999). *In vivo* selection of RNA-binding peptides from combinatorial libraries. Nucleic Acids Symp Ser, 213-214.
Huh, W. K., Falvo, J. V., Gerke, L. C., Carroll, A. S., Howson, R. W., Weissman, J. S. and O'Shea, E. K. (2003). Global analysis of protein localization in budding yeast. Nature *425*, 686-691.
Jain, K. K. (2000). Applications of proteomics in oncology. Pharmacogenomics *1*, 385-393.
Ji, Y., Marra, A., Rosenberg, M., and Woodnutt, G. (1999). Regulated antisense RNA eliminates alpha-toxin virulence in *Staphylococcus aureus* infection. J Bacteriol *181*, 6585-6590.
Ji, Y., Zhang, B., Van Horn, S. F., Warren, P., Woodnutt, G., Burnham, M. K. R., and Rosenberg, M. (2001). Identification of critical staphylococcal genes using conditional phenotypes generated by antisense RNA. Science *293*, 2266-2269.
Kay, B. K., Kasanov, J. and Yamabhai, M. (2001). Screening phage-displayed combinatorial peptide libraries. Methods *24*, 240-246.
Koizumi, M., Soukup, G. A., Kerr, J. N., and Breaker; R. R. (1999). Allosteric selection of ribozymes that respond to the second messengers cGMP and cAMP. Nat Struct Biol *6*, 1062-1071.
Lee, S. J., Boos, W., Bouche, J. P., and Plumbridge, J. (2000). Signal transduction between a membrane-bound transporter, PtsG, and a soluble transcription factor, Mlc, of *Escherichia coli.* EMBO J *19*, 5353-5361.
Len, A. C., Cordwell, S. J., Harty, D. W., and Jacques, N. A. (2003). Cellular and extracellular proteome analysis of *Streptococcus mutans* grown in a chemostat. Proteomics *3*, 627-646.
Lin, H. and Cornish, V. W. (2002). Screening and selection methods for large-scale analysis of protein function. Angew Chem Int Ed *41*, 4402-4425.
Mandal, M., Boese, B., Barrick, J. E., Winkler, W. C., and Breaker, R. R. (2003). Riboswitches control fundamental biochemical pathways in *Bacillus subtilis* and other bacteria. Cell *113*, 577-586.
Martzen, M. R., McCraith, S. M., Spinelli, S. L., Torres, F. M., Fields, S., Grayhack, E. J., and Phizicky, E. M. (1999). A biochemical genomics approach for identifying genes by the activity of their products. Science *286*, 1153-1155.
Mourez, M., Kane, R. S., Mogridge, J., Metallo, S., Deschatelets, P., Sellmann, B. R., Whitesides, G. M. and Collier, R. J. (2001). Designing a polyvalent inhibitor of anthrax toxin. Nat Biotechnol *19*, 958-961.
Müller, G., Hecht, B., Helbl, V., Hinrichs, W., Saenger, W. and Hillen, W. (1995). Characterization of non-inducible Tet repressor mutants suggests conformational changes necessary for induction. Nat Struct Biol *2*, 693-703.
Nakayama, H., Izuta, M., Nagahashi, S., Sihta, E. Y., Sato, Y., Yamazaki, T., Arisawa, M., and Kitada, K. (1998). A controllable gene-expression system for the pathogenic fungus *Candida glabrata.* Microbiology *144*, 2407-2415.
Neddermann, P., Gargioli, C., Muraglia, E., Sambucini, S., Bonelli, F., De Francesco, R., and Cortese, R. (2003). A novel, inducible, eukaryotic gene expression system based on the quorum-sensing transcription factor TraR. EMBO Rep *4*, 159-165.
Okinaka, Y., Yang, C. H., Perna, N. T., and Keen, N. T. (2002). Microarray profiling of *Erwinia chrysanthemi* 3937 genes that are regulated during plant infection. Mol Plant Microbe Interact *15*, 619-629.
Park, S. H. and Raines, R. T. (2000). Genetic selection for dissociative inhibitors of designated protein-protein interactions. Nat Biotechnol *18*, 847-851.
Peterson, R. D. and Feigon, J. (1996). Structural change in Rev responsive element RNA of HIV-1 on binding Rev peptide. J Mol Bio *264*, 863-877.
Phizicky, E., Bastiaens, P. I., Zhu, H., Snyder, M., and Fields, S. (2003). Protein analysis on a proteomic scale. Nature *422*, 208-215.
Rosenkrands, I., Slayden, R. A., Crawford, J., Aagaard, C., Barry, C. E., 3rd, and Andersen, P. (2002). Hypoxic response of *Mycobacterium tuberculosis* studied by metabolic labeling and proteome analysis of cellular and extracellular proteins. J Bacteriol *184*, 3485-3491.
Schnappinger, D., Schubert, P., Pfleiderer, K. and Hillen, W. (1998). Determinants of protein-protein recognition by four helix bundles: changing the dimerization specificity of Tet repressor. EMBO J *17*, 535-543.
Scholz, 0., Thiel, A., Hillen, W., and Niederweis, M. (2000). Quantitative analysis of gene expression with an improved green fluorescent protein. Eur J Biochem *267*, 1565-1570.
Schreiber, V., Steegborn, C., Clausen, T., Boos, W., and Richet, E. (2000). A new mechanism for the control of a prokaryotic transcriptional regulator: antagonistic binding of positive and negative effectors. Mol Microbiol *35*, 765-776.
Schubert, P., Schnappinger, D., Pfleiderer, K. and Hillen, W. (2001). Identification of a stability determinant on the edge of the Tet repressor four-helix bundle dimerization motif. Biochemistry *40*, 3257-3263.
Schwebach, J. R., Chen, B., Glatman-Freedman, A., Casadevall, A., McKinney, J. D., Harb, J. L., McGuire, P. J., Barkley, W. E., Bloom, B. R., and Jacobs, W. R., Jr. (2002). Infection of mice with aerosolized *Mycobacterium tuberculosis:* use of a nose-only apparatus for delivery of low doses of inocula and design of an ultrasafe facility. Appl Environ Microbiol *68*, 4646-4649.
Shimizu-Sato, S., Huq, E., Tepperman, J. M., and Quail, P. H. (2002). A light-switchable gene promoter system. Nat Biotechnol *20*, 1041-1044.
Smith, L. D. and Bertrand, K. P. (1998). Mutations in the Tn*10 tet* repressor that interfere with induction. Location of the tetracycline-binding domain. J Mol Bio *203*, 949-959.
Soukup, G. A., and Breaker, R. R. (1999). Engineering precision RNA molecular switches. Proc Natl Acad Sci USA *96*, 3584-3589.
Soukup, G. A., Emilsson, G. A., and Breaker, R. R. (2000). Altering molecular recognition of RNA aptamers by allosteric selection. J Mol Biol *298*, 623-632.
Staudinger, B. J., Oberdoerster, M. A., Lewis, P..J., and Rosen, H. (2002). mRNA expression profles for *Escherichia coli* ingested by normal and phagocyte oxidase-deficient human neutrophils. J Clin Invest *110*, 1151-1163.
Stormo, G. D. (2003). New tricks for an old dogma: riboswitches as *cis*-only regulatory systems. Mol Cell *11*, 1419-1420.
Sudarsan, N., Barrick, J. E., and Breaker, R. R. (2003). Metabolite-binding RNA domains are present in the genes of eukaryotes. RNA *9*, 644-647.
Suess, B., Hanson, S., Berens, C., Fink, B., Schroeder, R., and Hillen, W. (2003). Conditional gene expression by controlling translation with tetracycline-binding aptamers. Nucleic Acids Res *31*, 1853-1858.
Tang, J., and Breaker, R. R. (1997). Rational design of allosteric ribozymes. Chem Biol *4*, 453-459.
Tuerk, C. and Gold, L. (1990). Systematic evolution of ligands by exponential enrichment: RNA ligands to bacteriophage T4 DNA polymerase. Science *249*, 505-510.
Urlinger, S., Baron, U., Thellmann, M., Hasan, M. T., Bujard, H., and Hillen, W. (2000). Exploring the sequence space for tetracycline-dependent transcriptional activators: novel mutations yield expanded range and sensitivity. Proc Natl Acad Sci USA *97*, 7963-7968.
Uzzau, S., Figueroa-Bossi, N., Rubino, S., and Bossi, L. (2001). Epitope tagging of chromosomal genes in *Salmonella.* Proc Natl Acad Sci USA *98*, 15264-15269.
van Deursen, F. J., Shahi, S. K., CM, R. T., Hartmann, C., Guerra-Giraldez, C., Matthews, K. R., and Clayton, C. E. (2001). Characterisation of the growth and differentiation *in vivo* and *in vitro*-of bloodstream-form *Trypanosoma brucei* strain TREU 927. Mol Biochem Parasitol *112*, 163-171.
von Eggeling, F., Davies, H., Lomas, L., Fiedler, W., Junker, K., Claussen, U., and Ernst, G. (2000). Tissue-specific microdissection coupled with ProteinChip array technologies: applications in cancer research. Biotechniques *29*, 1066-1070.
Weber, W., Fux, C., Daoud-El Baba, M., Keller, B., Weber, C. C., Kramer, B. P., Heinzen, C., Aubel, D., Bailey, J. E., and Fussenegger, M. (2002). Macrolide-based transgene control in mammalian cells and mice. Nat Biotechnol *20*, 901-907.
Weber, W., Schoenmakers, R., Spielmann, M., El-Baba, M. D., Folcher, M., Keller, B., Weber, C. C., Link, N., van de Wetering, P., Heinzen, C., *et al.* (2003). Streptomyces-derived quorum-sensing systems engineered for adjustable transgene expression in mammalian cells and mice. Nucleic Acids Res *31*, e71.
Weeks, K. M., Ampe, C., Schultz, S. C., Steitz, T. A., and Crothers, D. M. (1990). Fragments of the HIV-1 Tat protein specifically bind TAR RNA. Science *249*, 1281-1285.
Werstuck, G., and Green, M. R. (1998). Controlling gene expression in living cells through small molecule-RNA interactions. Science *282*, 296-298.
Wilson, D. S., Keefe, A. D., Szostak, J. W. (2001). The use of mRNA display to select high-affinity protein-binding peptides. Proc Natl Acad Sci USA *98*, 3750-3755.
Winkler, W., Nahvi, A., and Breaker, R. R. (2002a). Thiamine derivatives bind messenger RNAs directly to regulate bacterial gene expression. Nature *419*, 952-956.
Winkler, W. C., Cohen-Chalamish, S., and Breaker, R. R. (2002b). An mRNA structure that controls gene expression by binding FMN. Proc Natl Acad Sci USA *99*, 15908-15913.
Winkler, W. C., Nahvi, A., Sudarsan, N., Barrick, J. E., and Breaker, R. R. (2003). An mRNA structure that controls gene expression by binding S-adenosylmethionine. Nat Struct Biol *10*, 10.
Wissmann, A., Baumeister, R., Müller, G., Hecht, B., Helbl, V., Pfleiderer, K. and Hillen, W. (1991). Amino acids determining operator binding specificity in the helix-turn-helix motif of *Tn10* Tet repressor. EMBO J *10*, 4145-4152.
Wray, L. V., Jr., Zalieckas, J. M., and Fisher, S. H. (2001). *Bacillus subtilis* glutamine synthetase controls gene expression through a protein-protein interaction with transcription factor TnrA. Cell *107*, 427-435.
Yao, F., Svensjö, T., Winkler, T., Lu, M., Eriksson, C., and Eriksson, E. (1998). Tetracycline repressor, *tetR*, rather than the tetR-mammalian cell transcription factor fusion derivatives, regulates inducible gene expression in mammalian cells. Hum Gene Ther *9*, 1939-1950.
Zhang, L., Fan, F., Palmer, L. M., Lonetto, M. A., Petit, C., Voelker, L. L., St John, A., Bankosky, B., Rosenberg, M., and McDevitt, D. (2000). Regulated gene expression in *Staphylococcus aureus* for identifying conditional lethal phenotypes and antibiotic mode of action. Gene *255*, 297-305.
Zhang, Q., Harada, K., Cho, H. S., Frankel, A. D., and Wemmer, D. E. (2001). Structural characterization of the complex of the Rev response element RNA with a selected peptide. Chem Biol *8*, 511-520.

## Claims

1. A method for monitoring the expression level of a gene in a host cell by modulating the activity of a regulatory biomolecule, comprising the steps of:
(a) transforming a cell expressing a regulatory biomolecule with a nucleic acid molecule comprising an open reading frame encoding an interaction partner of said biomolecule in expressible form, wherein
(i.) said regulatory biomolecule is either a nucleic acid binding molecule that effects its regulatory activity upon binding or an allosterically controlled ribonucleic acid molecule; and
(ii.) the interaction partner of the biomolecule is encoded by a nucleic acid molecule comprising:
(1) a nucleic acid sequence encoding a tagged (poly)peptide, or
(2) a nucleic acid sequence encoding a tagged (poly)peptide or a peptide tag, a selectable marker gene and additional nucleotide sequences for site specific, in-frame integration of said nucleic acid molecule into the coding sequence of at least one host (poly)peptide of interest,
wherein said tag comprises the interacting residues of the interaction partner
and
(b) assessing the expression level of the gene.

2. The method of claim 1, wherein the activity or degree of modulation of the activity of the biomolecule is measured via a readout system.

3. The method of claim 2, wherein
(a) the readout system is provided by a nucleic acid molecule encoding a reporter protein;
(b) the regulatory biomolecule is
(i.) a nucleic acid binding (poly)peptide selected from the group consisting of regulators of transcription, regulators of translation, recombinases, (poly)peptides involved in RNA transport,
(ii.) an RNA molecule selected from the groups consisting of allosteric ribozymes, riboswitches or translation-regulating aptamers,
(iii.)
and
(c) the nucleic acid binding biomolecule is allosterically regulated.

4. The method of claim 2 or 3 wherein the nucleic acid binding (poly)peptide comprises the (poly)peptide sequence of
(a) Tetracycline repressor;
(b) Lac repressor;
(c) Xylose repressor;
(d) AraC protein
(e) TetR-based T-Rex system;
(f) Erythromycin-specific repressor MphR(A);
(g) Pip (pristinamycin interacting protein);
(h) ScbR of *Streptomyces coelicolor*;
(i) TraR of *Agrobacterium tumefaciens;* fused to the eukaryotic activation domain p65 of NFκB;
(j) chimeric proteins consisting of the:
(i.) Gal4 DNA-binding domain and either a full-length PhyA protein (PhyA-GBD) or the N-terminal photosensory domain of PhyB [PhyB(NT)-GBD] of *Arabidopsis thaliana*;
(ii.) a steroid hormone regulated system consisting of (1) a Gal4 DNA-binding domain fused to a human progesterone receptor ligand binding domain and an NFκB-derived p65 eukaryotic transcription activation domain and (2) the inducer mifepristone
(iii.) a dimerizer system consisting of (1) a ZFHD1 DNA-binding domain fused to FKBP 12, (2) FRAP fused to the NFκB-derived p65 activation domain and (3) rapamycin, AP22565 or AP12967 as heterodimer-forming agents, or
(iv.) an Ecdysone-Inducible Expression System containing (1) a modified form of the *Drosophila* ecdysone receptor (VgEcR) fused to a VP16 activation domain, (2) the mammalian homologue RXR of *ultraspiracle,* the natural binding partner of the ecdysone receptor in *Drosophila* and (3) the inducers ponasterone A and muristerone A:

5. The method of claim 3 or 4, wherein the reporter protein of the readout system is ß-galactosidase, CAT, ß-glucuronidase, ß-xylosidase, XylE (catechol dioxygenase), TreA (trehalase), GFP and variants CFP, YFP, EGFP, GFP+ , bacterial luciferase (*luxAB*), *Photinus* luciferase; *Renilla* luciferase, coral-derived photoproteins including DsRed, HcRed, AmCyan, ZsGreen, ZsYellow, AsRed, alkaline phosphatase or secreted alkaline phosphatase.

6. The method of claim 3 or 4, wherein the reporter protein is a protein that confers resistance to an antibiotic.

7. The method of any one of claims 1 to 6, wherein the cell is selected from a mammalian, insect, nematode, plant, yeast, protist cell, gram-positive or gram-negative bacteria, archaebacteria or protozoa.

8. The method of any one of claims 1 to 7, wherein all or a subset of the proteins encoded by the cell are tagged.

9. A method of producing and/or selecting a compound capable of modulating the activity of a nucleic acid binding protein comprising the steps of:
(a) conducting a selection of compounds with the nucleic acid binding target protein under conditions allowing an interaction of the compound and the nucleic acid binding protein;
(b) removing unspecificly bound compounds;
(c) detecting specific binding of compounds to the nucleic acid binding target protein;
(d) expressing in a cell, the nucleic acid binding protein and providing in trans the coding sequence of at least one indicator gene, wherein said coding sequence is under control of the target sequence of the nucleic acid binding protein;
(e) adding a candidate compound to the cell of step (d);
(f) determining the amount or activity of the indicator protein, wherein a reduced or increased amount of indicator protein is indicative of compounds, capable of modulating the activity of the nucleic acid binding protein; and
(g) selecting compounds capable of modulating the activity of the nucleic acid binding protein.

10. A nucleic acid molecule encoding a (poly)peptide comprising the sequence
(a) 
Met – Trp – Thr – Trp – Asn – Ala – Tyr – Ala – Phe – Ala – Ala – Pro
– Ser – Gly – Gly – Gly – Ser (SEQ ID NO: 1);
(b) 
Trp – Thr – Trp – Asn – Ala – Tyr – Ala – Phe – Ala – Ala – Pro – Ser
– Gly – Gly – Gly – Ser (SEQ ID NO: 2);
(c) 
Trp – Thr – Trp – Asn – Ala – Tyr – Ala – Phe – Ala – Ala – Pro – Ser
(SEQ ID NO: 3);
(d) 
Ser – Gly – Gly – Ala - Trp – Thr – Trp – Asn – Ala – Tyr – Ala – Phe –
Ala – Ala – Pro – Ser – Gly – Gly – Gly – Ser (SEQ ID NO: 4);
(e) 
Ser – Gly – Gly – Ala - Trp – Thr – Trp – Asn – Ala – Tyr – Ala – Phe –
Ala – Ala – Ala – Ser – Gly – Gly – Gly – Ser (SEQ ID NO: 5);
(f) 
Ser – Gly – Gly – Ala - Trp – Thr – Trp – Asn – Ala – Tyr – Ala – Phe –
Ala – Ala – Pro – Ala – Gly – Gly – Gly – Ser (SEQ ID NO: 6);
(g) 
Ser – Gly – Gly – Ala - Trp – Thr – Trp – Asn – Ala – Tyr – Ala – Phe –
Ala – Ala – Pro – Ser – Gly – Arg – Gly – Ser (SEQ ID NO: 7);
(h) 
Ser – Gly – Gly – Ala - Trp – Thr – Trp – Asn – Ala – Tyr – Ala – Phe –
Ala – Ala – Pro – Ser – Asp – Gly – Gly – Leu (SEQ ID NO: 8);
(i) 
Ser – Gly – Gly – Ala - Trp – Thr – Trp – Asn – Ala – Tyr – Ala – Phe –
Ala – Ala – Pro – Ser – Gly – Glu – Gly – Ser (SEQ ID NO: 9);
(j) 
Ser – Gly – Gly – Ala - Trp – Thr – Trp – Asn – Ala – Tyr – Ala – Phe –
Ala – Ala – Pro – Ser – Gly – Gly – Gly – Trp (SEQ ID NO: 10);
(k) 
Ser – Gly – Gly – Ala - Trp – Thr – Trp – Asn – Ala – Tyr – Ala – Phe –
Ala – Ala – Pro – Ser – Gly – Gly – Cys – Ser (SEQ ID NO: 11);
(I) 
Ser – Gly – Gly – Ala - Trp – Thr – Trp – Asn – Ala – Tyr – Ala – Phe –
Ala – Ala – Pro – Ser – Gly – Gly – Asp – Ser (SEQ ID NO: 12);
(m) 
Ser – Gly – Gly – Ala - Trp – Thr – Trp – Asn – Ala – Tyr – Ala – Phe –
Ala – Ala – Pro – Ser – Gly – Gly – Arg – Ser (SEQ ID NO: 13);
(n) 
Ser – Gly – Gly – Ala - Trp – Thr – Trp – Asn – Ala – Phe – Ala – Phe
– Ala – Ala – Pro – Ser – Gly – Gly – Gly – Ser (SEQ ID NO: 14); or
(o) a nucleic acid molecule, the complementary strand of which hybridizes under stringent conditions to the nucleic acid molecule of any one of (a) to (n), wherein said nucleic acid molecule encodes an interaction partner which is capable of modulating the activity of a nucleic acid binding protein.

11. A (poly)peptide encoded by the nucleic acid sequence of claim 10.

12. An expression vector, comprising an expression control sequence, a multiple cloning site for inserting a gene of interest and the nucleic acid molecule of claim 10, wherein the gene of interest is inserted in-frame with the ORF encoding the peptide.

13. A host cell containing the nucleic acid molecule or the expression vector of claim 12.

14. The host cell of claim 13, wherein the nucleic acid molecule is fused in frame to at least one chromosomal sequence encoding a (poly)peptide.

15. The host cell of claim 13 or 14, also containing
(a) the coding sequence of a reporter protein which is under control of a nucleic acid binding protein; and
(b) the coding sequence of the nucleic acid binding protein of (a),
wherein said coding sequences are operatively linked to expression control sequences.

16. The host cell of any one of claims 13 to 15, wherein the nucleic acid binding protein is a repressor of transcription.

17. The host cell of claim 16, wherein the nucleic acid binding protein is a (poly)peptide comprising the (poly)peptide sequence of
(a) Tetracycline repressor;
(b) Lac repressor.
(c) Xylose repressor;
(d) AraC protein
(e) TetR-based T-Rex system;
(f) Erythromycin-specific repressor MphR(A);
(g) Pip (pristinamycin interacting protein);
(h) ScbR of *Streptomyces coelicolor*;
(i) TraR of *Agrobacterium tumefaciens* fused to the eukaryotic activation domain p65 of NFκB; or
(j) chimeric proteins consisting of the:
(i.) Gal4 DNA-binding domain and either (1) a full-length PhyA protein (PhyA-GBD) or (2) the N-terminal photosensory domain of PhyB [PhyB(NT)-GBD] of *Arabidopsis thaliana*;
(ii.) a steroid hormone regulated system consisting of (1) a Gal4 DNA-binding domain fused to a human progesterone receptor ligand binding domain and an NFκB-derived p65 eukaryotic transcription activation domain and (2) the inducer mifepristone;
(iii.) a dimerizer system consisting of (1) a ZFHD1 DNA-binding domain fused to FKBP 12, (2) FRAP fused to the NFκB-derived p65 activation domain and (3) rapamycin, AP22565 or AP12967 as heterodimer-forming agents;
(iv.) an Ecdysone-Inducible Expression System containing (1) a modified form of the *Drosophila* ecdysone receptor (VgEcR) fused to a VP16 activation domain, (2) the mammalian homologue RXR of *ultraspiracle,* the natural binding partner of the ecdysone receptor in *Drosophila* and (3) the inducers ponasterone A and muristerone A.

18. The host cell of any one of claims 15 to 17, wherein the nucleic acid binding protein is an enhancer or activator of transcription.

19. An ensemble of host cells of any one of claims 13 to 18, wherein said ensemble comprises two or more cells, each of which contain at least one nucleic acid molecule fused in frame to an open reading frame encoding a (poly)peptide.

20. The ensemble of host cells of claim 19, wherein said ensemble contains subpopulations with different open reading frames being fused to said nucleic acid molecule.

21. The ensemble of host cells of claim 20, wherein the sum of said open reading frames forms the proteome of the host cell.

22. A non-human animal comprising the host cell of any one of claims 13 to 18 or the ensemble of any one of claims 19 to 21.

23. A kit comprising
(a) the nucleic acid molecule of claim 10;
(b) the (poly)peptide of claim 11;
(c) the vector of claim 12;
(d) the host cell or ensemble of host cells of any one of claims 13 to 22; and/or
(e) instructions for use;
in one or more containers.

24. Use of the (poly)peptide of claim 11, the nucleic acid molecule of claim 10, the expression vector of claim 12 or the host cell or ensemble of host cells of any one of claims 13 to 22 for monitoring expression of a gene.
